# EUROPÄISCHE PATENTSCHRIFT

(11) **EP 3 408 370 B1**
(45) Veröffentlichungstag und Bekanntmachung des Hinweises auf die Patenterteilung: **07.04.2021**
(21) Anmeldenummer: 17701548.4
(22) Anmeldetag: 27.01.2017
(51) Int. Cl.: C12M 1/00, C12M 3/00, C12M 1/12, C12M 1/34

(54) **EINWEG-ANSCHLUSSEINRICHTUNG**
DISPOSABLE CONNECTION DEVICE
DISPOSITIF DE CONNEXION A UNE VOIE

(30) Priorität: 29.01.2016 EP 16153294
(43) Veröffentlichungstag der Anmeldung: 05.12.2018
(73) Patentinhaber: Eppendorf AG, 22339 Hamburg (DE)
(72) Erfinder: ARNOLD, Matthias, 52074 Aachen (DE); SELZER, Sebastian, 52074 Aachen (DE); EIKELMANN, Sven, 32469 Petershagen (DE); BEESE, Jochen, 22848 Norderstedt (DE)
(74) Vertreter: Eisenführ Speiser
(86) Internationale Anmeldenummer: PCT/EP2017/051865
(87) Internationale Veröffentlichungsnummer: WO 2017/129800

(56) Entgegenhaltungen:
- EP-A1- 2 251 407
- EP-A1- 2 674 479
- EP-A1- 2 853 584
- WO-A1-2008/016411
- WO-A1-2013/053779
- WO-A1-2015/077663
- WO-A2-2011/126793
- DE-A1- 2 137 854
- DE-A1-102004 059 146
- DE-U1-202009 015 434
- US-A1- 2008 131 957

## Beschreibung

Die Erfindung betrifft eine Einweg-Anschlusseinrichtung zum Einsetzen in eine Anschlussöffnung eines Bioreaktors, insbesondere in eine Anschlussöffnung einer Kopfplatte eines formstabilen Bioreaktors und/oder in eine Anschlussöffnung eines Anschlusselements eines Beutel-Bioreaktors. Ferner betrifft die Erfindung eine Kopfplatte für einen Bioreaktor sowie einen Bioreaktor zur Kultivierung von Mikroorganismen und/oder Zellkulturen. Darüber hinaus ferner betrifft die Erfindung ein Anschlusselement für einen Beutel-Bioreaktor sowie einen Beutel-Bioreaktor zur Kultivierung von Mikroorganismen und/oder Zellkulturen.

Ein Mikroorganismus ist ein mikroskopisch kleiner, meist einzelliger oder wenigzelliger, pflanzlicher oder tierischer Organismus. Mikroorganismen können daher auch als die Gesamtheit aller nicht mit bloßem Auge erkennbaren Organismen bezeichnet werden. Dazu gehören verschiedene Gruppen der Viren, Bakterien, Archaeen, Protozoen, Pilze und Mikro-Algen. Eine Vielzahl an Mikroorganismen wird als nützlich angesehen, da diese beispielsweise für geochemische Stoffkreisläufe vorteilhaft sind, zur Produktion von bestimmten Nahrungsmitteln verwendet und als Produzenten von Arzneimitteln eingesetzt werden. Ferner werden häufig pathogene Mikroorganismen zu Forschungszwecken gezüchtet. Die Kultivierung von Mikroorganismen beinhaltet unter anderem die Schaffung und Aufrechterhaltung von Bedingungen, die ein Wachstum der Organismen gewährleistet, wobei darüber hinaus häufig das Ziel einer Vermehrung der Organismen verfolgt wird.

Eine Zellkultur ist die Kultivierung einer Zellpopulation außerhalb eines Organismus' unter kontrollierten Bedingungen, wobei die physiologischen Prozesse der Zellen vollständig oder überwiegend aufrechterhalten werden. Die Kultivierung enthält neben der Zellpopulation daher unter anderem ein Kulturmedium, das das Wachstum, die Proliferation und die Differenzierung der Zellkultur ermöglicht. Üblicherweise umfassen die Kulturmedien Aminosäuren, Vitamine, anorganische Salze und Puffergemische sowie in der Regel Glutamin. Zellkulturen werden für unterschiedlichste Zwecke eingesetzt, wobei der Einsatzschwerpunkt im Bereich der Forschung und Entwicklung liegt. Für die Herstellung einer Vielzahl von biotechnischen Produkten haben Zellkulturen ebenfalls eine hohe Bedeutung.

Die Kultivierung von Mikroorganismen und/oder Zellkulturen wird in der Regel unter sterilen Bedingungen im Kulturmedium, das auch als Kulturbrühe bezeichnet wird, in einem Reaktionsraum im Inneren eines Bioreaktors durchgeführt. Zumindest während eines bestimmten Zeitraums der Kultivierung werden dem System zur Aufrechterhaltung der biologischen Vorgänge gasförmige und/oder flüssige Fluide zugeführt und/oder gasförmige und/oder flüssige Fluide abgeführt, welche auch, durch biologische Vorgänge erzeugte, weitere Bestandteile enthalten können.

Bioreaktoren, die auch als Fermentationsreaktoren oder als Fermenter bezeichnet werden, umfassen insbesondere einen Behälter, in dessen Inneren unter regulierten und definierten Bedingungen biologische bzw. biotechnologische Prozesse ablaufen bzw. bestimmte Kulturen, insbesondere Mikroorganismen und/oder Zellkulturen, kultiviert werden sollen. Das Innere des Bioreaktors und/oder des Behälters wird auch als Reaktionsraum bezeichnet. Der Behälter weist in der Regel eine vollständig oder überwiegend geschlossene Umwandung auf, wobei eine Seite auch von einer Kopfplatte abgeschlossen sein kann. Der Behälter und/oder die Kopfplatte weisen ferner Anschlussöffnungen auf, die der Aufnahme bzw. der Befestigung von Anschlüssen dienen. Anschlüsse dienen als Verbindungselement zwischen dem Innenraum des Behälters des Bioreaktors und außerhalb des Behälters verorteten Zuleitungen, wobei diese Zuleitungen dem Zuleiten von gasförmigen und/oder flüssigen Fluiden oder der Zuführung von Festkörpern, beispielsweise Sensoren, in den Innenraum des Behälters dienen.

Bioreaktoren sind häufig aus Glas und/oder Metall, insbesondere rostfreiem Stahl, ausgebildet. Da ein einzelner Bioreaktor für unterschiedliche biologische bzw. biotechnologische Prozesse eingesetzt werden kann und zwischen den unterschiedlichen Prozessen in der Regel eine Sterilisation stattfinden muss, sind die Materialien Glas und/oder Metall besonders geeignet, da diese Materialien gut zu sterilisieren sind. Diese Sterilisation findet vorzugsweise durch eine Heißdampfsterilisation in einem Autoklaven statt. Der Sterilisations- und Reinigungsprozess kann ferner einer Validierung unterliegen und seine Durchführung ist für jeden einzelnen Bioreaktor zu dokumentieren.

Die Rückstände in einem nicht vollständig sterilisierten Bioreaktor können den im Anschluss durchgeführten biologischen bzw. biotechnologischen Prozess verfälschen und die Ergebnisse somit unbrauchbar machen. Die Verfälschung des Prozesses durch Rückstände gilt einerseits für Rückstände innerhalb des Reaktors, andererseits auch für Rückstände in Vorrichtungen, die im unmittelbaren und/oder mittelbaren Kontakt zum Reaktionsraum des Bioreaktors stehen. Hierzu zählen insbesondere, aber nicht ausschließlich, Anschlüsse, über die Primär- und Sekundärstoffe sowie Instrumente, wie beispielsweise Sensoren, in den Reaktionsraum eingebracht werden können. Zusätzlich oder alternativ können an diese Anschlüsse Fluidleitungen angebracht werden, die als Zu- oder Ableitungen ausgebildet sein können.

EP2674479 beschreibt einen Einwegbioreaktor mit einer Kopfplatte mit Tauchrohren. Die Tauchrohre, ragen in den Reaktionsraum und können verschiedene Instrumente, Sensoren oder Leitungen aufnehmen und korrespondieren mit Anschlüssen auf der Außenseite der Kopfplatte. Vorzugsweise zwei der Anschlüsse können als Schraubanschlüsse mit Innengewinde ausgebildet sein, wodurch an den Anschlüssen Schraubverbindungen hergestellt werden können. Das Material des gesamten Bioreaktors ist dampf-sterilisierbar und sowohl die Kopfplatte als auch die Anschlüsse sind als Einwegartikel konzipiert.

Diese Anschlüsse sind häufig an einer Kopfplatte des Bioreaktors verortet. Die Kopfplatte weist eine flächige Geometrie auf, wobei die Fläche vorzugsweise eine im Wesentlichen horizontale Erstreckung aufweist. Die Anschlüsse werden in der Regel an oder in Öffnungen der Kopfplatte angebracht. Vorzugsweise weisen diese Öffnungen eine im Wesentlichen vertikale Durchtrittsrichtung auf.

Die aufwendige Sterilisation und gegebenenfalls zusätzliche Validierung selbiger kann durch den Einsatz sogenannter Einwegbioreaktoren bzw. single-use Bioreaktoren vermieden werden. Diese sind für die Durchführung lediglich eines einzelnen biologischen bzw. biotechnologischen Prozesses ausgebildet und werden im Anschluss an diese Durchführung entsorgt. Durch die Bereitstellung eines neuen, vorzugsweise im Herstellungsprozess sterilisierten, Einwegbioreaktors für jeden Prozess kann das Risiko einer (Kreuz-) Kontamination reduziert werden und gleichzeitig entfällt der Aufwand der Durchführung und Dokumentation einer einwandfreien Reinigung und/oder Sterilisation eines zuvor genutzten Bioreaktors. Einwegbioreaktoren sind oft als vollständig oder teilweise nicht formstabile Behälter ausgebildet, beispielsweise als Beutel-Biorektoren und/oder als Behälter mit zumindest abschnittsweise nicht formstabilen Wänden.

Ein steriler Einwegbioreaktor umfasst in der Regel einen oder mehrere Anschlüsse über die Primär- und Sekundärstoffe, beispielweise gasförmige oder flüssige Fluide, sowie verschiedene Festkörper, wie beispielsweise Sensoren, in den Reaktionsraum eingebracht werden können.

Anschlüsse der zuvor genannten Art haben eine Vielzahl an technischen Anforderungen, die zudem Interdependenzen aufweisen. Die zwingende Voraussetzung einer sterilen Umgebung umfasst zusätzlich zum Innenraum des Behälters des Bioreaktors zusätzlich alle Zuleitungen zu diesem Innenraum. Dabei sind insbesondere auch die Anschlüsse von dieser Voraussetzung eingeschlossen, da die Anschlüsse einen direkten Kontakt zum Innenraum haben und/oder Medien oder Sonstiges leiten, die in den Innenraum gelangen. Ferner ist es eine Anforderung, dass der Anschluss den Innenraum des Bioreaktors im Wesentlichen hermetisch von der Umgebung außerhalb des Bioreaktors abkoppelt. Die existierenden Vorrichtungen bieten verschiedene Vorteile, jedoch sind weitere Verbesserungen wünschenswert.

Es ist daher eine Aufgabe der vorliegenden Erfindung, eine Einweg-Anschlusseinrichtung zum Einsetzen in eine Anschlussöffnung eines Bioreaktors, eine Kopfplatte für einen Bioreaktor sowie einen Bioreaktor zur Kultivierung von Mikroorganismen und/oder Zellkulturen bereitzustellen, welche ein oder mehrere der genannten Nachteile vermindern oder beseitigen. Insbesondere ist es eine Aufgabe der Erfindung, eine Einweg-Anschlusseinrichtung zum Einsetzen in eine Anschlussöffnung eines Bioreaktors, eine Kopfplatte für einen Bioreaktor sowie einen Bioreaktor zur Kultivierung von Mikroorganismen und/oder Zellkulturen bereitzustellen, welche das Kontaminationsrisiko reduzieren und dennoch eine kostengünstige Lösung bereitstellen.

Die erfindungsgemäße Aufgabe wird gemäß den Ansprüchen 1, 10, 12 und 13 gelöst durch eine Einweg-Anschlusseinrichtung eingesetzt in eine Anschlussöffnung einer Kopfplatte eines Bioreaktors und/oder in eine Anschlussöffnung eines Beutel-Bioreaktors, mit mehreren Durchgängen und einem Befestigungsabschnitt, dadurch gekennzeichnet, dass die Einweg-Anschlusseinrichtung einstückig und aus Kunststoff ausgebildet ist und der Befestigungsabschnitt an einer Außenumfangsfläche eine Befestigungsstruktur zum Befestigen der Einweg-Anschlusseinrichtung in der Anschlussöffnung aufweist.

Die Einweg-Anschlusseinrichtung wird erfindungsgemäß in eine Anschlussöffnung einer Kopfplatte eines Bioreaktors und/oder in eine Anschlussöffnung eines Beutel-Bioreaktors eingesetzt. Das Einsetzen bedeutet insbesondere, dass die Einweg-Anschlusseinrichtung in eine Anschlussöffnung eingebaut wird, was vorzugsweise das Einbetten und/oder Einfügen und/oder Einpassen der Einweg-Anschlusseinrichtung in und/oder an die zuvor bezeichnete Öffnung umfasst. Das Einsetzen kann ferner das lösbare oder nicht lösbare Befestigen der Einweg-Anschlusseinrichtung an einem Bestandteil eines Bioreaktors, insbesondere in der Anschlussöffnung, umfassen.

Anschlüsse können vielfach die Ursache für Kontaminationen des Innenraums des Bioreaktors sein. Die Ursache kann beispielsweise eine Kontamination der durch den Anschluss verlaufenden Durchleitungen oder eine nicht vollständige Abdichtung zwischen der äußeren Wandung des Anschlusses und einer Wandung bildende Öffnung im Behälter des Bioreaktors sein. Der Erfindung liegt daher die Erkenntnis zugrunde, dass die Sterilität im Bioreaktor neben diesem selbst ebenfalls und gerade von den Anschlüssen und deren meist komplexer Geometrie beeinflusst wird. Daher ist die erfindungsgemäße Einweg-Anschlusseinrichtung als ein Einwegbauteil ausgebildet. Ein Einwegbauteil wird im vorliegenden Anwendungsfall lediglich für eine einzige Anwendung eingesetzt und anschließend entsorgt. Die Einweg-Anschlusseinrichtung wird demnach für eine einzige Kultivierung von Mikroorganismen und/oder Zellkulturen eingesetzt. Dies reduziert das Kontaminationsrisiko erheblich und es ist keine Reinigung der Einweg-Anschlusseinrichtung notwendig. Ferner entfällt der gegebenenfalls durchzuführende Validierungsprozess.

Die bezeichnete Anschlussöffnung einer Kopfplatte eines Bioreaktors und/oder eines Anschlusselements eines Beutel-Bioreaktors dient der Aufnahme der Einweg-Anschlusseinrichtung. Vorzugsweise ist eine Längsachse in axialer Richtung der Einweg-Anschlusseinrichtung parallel zu einer Durchtrittsrichtung der Anschlussöffnung. Der Querschnitt, orthogonal zur Durchtrittsrichtung, der Anschlussöffnung ist vorzugsweise im Wesentlichen kreisrund, abgesehen von einer gegebenenfalls vorhandenen Befestigungsstruktur, die vorzugsweise komplementär zur Befestigungsstruktur der Einweg-Anschlusseinrichtung ausgebildet ist. Ferner vorzugsweise weist dieser Querschnitt der Anschlussöffnung einen schlitzförmigen, drei- oder mehreckigen Querschnitt auf. Die Geometrie und die Abmaße des Querschnitts sind vorzugsweise entlang der Durchtrittsrichtung variabel.

Die Anschlussöffnung und der Abschnitt der Einweg-Anschlusseinrichtung, der im Kontakt zu einer Wandung oder anderweitigen Begrenzung der Anschlussöffnung steht, sind vorzugsweise derart ausgebildet, dass durch den Kontakt der zwei Komponenten eine hermetische und/oder fluiddichte Versiegelung der Anschlussöffnung erfolgen kann, wobei vorzugsweise auch eine weiter unten beschriebene umlaufende Dichtung zum Einsatz kommen kann.

Der Bioreaktor umfasst einen Behälter und eine Kopfplatte. Der Behälter weist vorzugsweise eine Umwandung hinsichtlich seiner horizontalen Erstreckungen auf. Der Behälter weist ferner vorzugsweise eine Umwandung hinsichtlich einer vertikalen Erstreckung auf, wobei diese Umwandung durch einen Behälterboden gebildet werden kann. Die Kopfplatte als weiteres Element des Bioreaktors hat die Funktion, den Bioreaktor zu verschließen. Vorzugsweise weist die Kopfplatte eine im Wesentlichen flächige Geometrie auf, wobei die Fläche vorzugsweise eine überwiegend horizontale Erstreckung aufweist. Die Kopfplatte weist ferner mindestens eine Anschlussöffnung auf.

In einer weiteren bevorzugten Ausführungsvariante ist der Bioreaktor als ein sogenannter Beutel-Bioreaktor ausgeführt. Ein Beutel-Bioreaktor ist als vollständig flexibler Behälter oder teilweise nicht formstabiler Behälter ausgebildet, beispielsweise als Behälter mit zumindest abschnittsweise nicht formstabilen Umwandungen. Beutel-Bioreaktoren werden im Allgemeinen als Einwegbioreaktoren ausgebildet, so dass eine aufwendige Sterilisation und gegebenenfalls eine umfangreiche Validierung selbiger vermieden werden kann. Der Beutelbioreaktor umfasst ein Anschlusselement mit einer Anschlussöffnung.

Der Bioreaktor umfasst eine oder mehrere Anschlussöffnungen zur Anbringung von Einweg-Anschlusseinrichtungen über die Primär- und Sekundärstoffe, beispielweise gasförmige oder flüssige Fluide, sowie verschiedene Festkörper, wie beispielsweise Sensoren, in den Innenraum des Bioreaktors eingebracht werden können. Damit die Primär- und Sekundärstoffe und/oder Festkörper in den Innenraum des Bioreaktors eingebracht werden können, weisen die Einweg-Anschlusseinrichtungen erfindungsgemäß mehrere Durchgänge auf. Die Durchgänge sind vorzugsweise umwandet ausgebildet und können verschiedene Instrumente, Sensoren, Schläuche oder Leitungen aufnehmen.

Die Durchtrittsrichtung der mehreren Durchgänge ist vorzugsweise im Wesentlichen parallel zur Durchtrittsrichtung der Anschlussöffnung, in die die Einweg-Anschlusseinrichtungen eingefügt wird. Ferner vorzugsweise weisen die mehreren Durchgänge eine Durchtrittsrichtung auf, die im Wesentlichen parallel zu einer Längsachse der Einweg-Anschlusseinrichtung ist. Die mehreren Durchgänge weisen jeweils ein erstes Durchgangsende und ein zweites Durchgangsende auf. Zwischen diesen zwei Enden ist der Durchgang vollständig umwandet, so dass ein eintretendes Fluid am ersten Durchgangsende ausschließlich am zweiten Durchgangsende entweichen kann. Die axiale Länge der Durchgänge kann variieren. Mehrere Durchgänge sind mindestens zwei Durchgänge, vorzugsweise jedoch drei oder mehr Durchgänge.

Vorzugsweise korrespondieren die Durchgänge auf der Innenseite, der dem Bioreaktor zugewandten Seite, der Kopfplatte bzw. des Anschlusselements mit Vorrichtungen auf der Außenseite, der dem Bioreaktor abgewandten Seite, der Kopfplatte bzw. des Anschlusselements, sodass durch die Durchgänge die gasförmigen und/oder flüssigen Fluide und/oder Festkörper in den Reaktionsraum ein bzw. aus diesem herausgeführt werden können. Die Durchgangsenden auf der dem Bioreaktor abgewandten Seite sind demnach vorzugsweise ausgebildet zum Anschluss von Leitungen zur Ver- und/oder Entsorgung des Kulturmediums, zum Bereitstellen von Instrumenten, beispielsweise Sensorik, und/oder für weitere zur Durchführung der Kultivierung von Mikroorganismen und/oder Zellkulturen eingesetzten Betriebs- und Hilfsmittel und/oder -stoffe.

Vorzugsweise ragen die Durchgänge, zumindest mindestens einer der Durchgänge, so weit in den Reaktionsraum des Bioreaktors hinein, dass sie bei einer bestimmungsgemäßen Verwendung des Bioreaktors in einen im Reaktionsraum angeordneten Inhalt, beispielsweise eine Flüssigkeit, eintauchen. Die Durchgänge werden in diesem Fall auch als Tauchrohre bezeichnet. Dies hat den Vorteil, dass an der dem Innenraum des Bioreaktors zugewanden Seite der Einweg-Anschlusseinrichtung, keine Rohre oder Schläuche angeschlossen werden müssen, sondern die bereits an der Einweg-Anschlusseinrichtung ausgebildeten Durchgänge bzw. deren Umwandungen verwendet werden können. Zur Befestigung von weiteren Anschlüssen an den Durchgängen, insbesondere auf einer dem Bioreaktor abgewandten Seite der Einweg-Anschlusseinrichtung, wird vorzugsweise ein Luer-Lock-System vorgesehen.

Die Querschnitte, orthogonal zu der Durchtrittsrichtung, der mehreren Durchgänge sind vorzugsweise kreisrund. Ferner vorzugsweise können die Querschnitte der mehreren Durchgänge unterschiedliche Geometrien aufweisen. Die Durchmesser der mehreren Durchgänge können jeweils gleich oder unterschiedlich sein. Vorzugsweise beträgt ein Durchmesser eines einzelnen kreisrunden Durchgangs 1 mm bis 4 mm, ferner vorzugsweise 1,5 bis 2,5 mm und ferner vorzugsweise 2 mm.

Der Erfindung liegt die Erkenntnis zugrunde, dass die Zusammenfassung von mehreren Durchgängen in einer Einweg-Anschlussvorrichtung mit einem Befestigungsabschnitt, der an einer Außenumfangsfläche eine Befestigungsstruktur umfasst, gleich mehrere Vorteile vereint. Die Befestigungsstruktur dient dem Befestigen der Einweg-Anschlusseinrichtung in der Anschlussöffnung der Kopfplatte des Bioreaktors und/oder in der Anschlussöffnung des Beutel-Bioreaktors und ermöglicht so die sichere, dichte, schnelle und einfache Montage von mehreren Durchgängen am Bioreaktor. Durch die Ausbildung unterschiedlicher Varianten von Einweg-Anschlusseinrichtungen wird es so möglich, einerseits Mehrweg-Bioreaktoren mit unterschiedlichen Anschlüssen für unterschiedliche Anwendungen zu versehen, wobei auf aufwendige Sterilisationen verzichtet werden kann, und andererseits auch Einweg-Bioreaktoren durch Montage von unterschiedlichen Einweg-Anschlusseinrichtungen einfach und kostengünstig für verschiedene Anwendungen bereitzustellen. Die Zusammenfassung mehrere Durchgänge in der Einweg-Anschlusseinrichtung ermöglicht es zudem, die übrigen Komponenten von Einweg-Bioreaktoren mit möglichst wenig Varianten zu fertigen und somit die Kosten weiter zu senken. Durch die Ausbildung einer geeigneten Befestigungsstruktur auf der Außenoberfläche am Befestigungsabschnitt kann ferner - insbesondere je nach Anwendung mit Einweg- oder Mehrweg-Bioreaktoren - eine nicht lösbare oder eine lösbare Befestigung in der Anschlussöffnung der Kopfplatte bzw. des Anschlusselements vorgesehen werden.

Die Befestigungsstruktur kann das Befestigen durch unterschiedliche technische Lösungen gewährleisten. Vorzugsweise ist eine Rippenstruktur und/oder ferner vorzugsweise eine Schnappfingerstruktur und/oder ferner vorzugsweise eine Rastverbindung und/oder ferner vorzugsweise eine Clipverbindung und/oder ferner vorzugsweise ein Außengewinde vorgesehen. Die Befestigungsstruktur kann auch in Form eines derart bemessenen Außendurchmessers des Befestigungsabschnitts realisiert sein, dass eine Klemmverbindung bzw. ein Presssitz der Einweg-Anschlusseinrichtung in der Anschlussöffnung realisiert wird. Die Anschlussöffnung ist dahingehend ausgebildet mit einer oder mehreren der zuvor genannten Befestigungsstrukturen eine lösbare oder unlösbare Verbindung einzugehen. Beispielsweise weist die Anschlussöffnung im Falle eines Außengewindes an der Einweg-Anschlusseinrichtung ebenfalls ein Gewinde auf, um eine Schraubverbindung zu realisieren. Vorzugsweise können im Befestigungsabschnitt zusätzlich Dichtungsmittel und/oder Dichtungseinrichtungen eingebracht werden.

Der Erfindung liegt ferner die Erkenntnis zugrunde, dass eine einstückige und/oder einteilige Einweg-Anschlusseinrichtung verschiedene Vorteile bietet. Vorteilhaft ist beispielsweise die geringere Anzahl an Trennfugen bei der einstückigen Ausbildung. Zudem sinken die Toleranzanforderungen, da aufgrund von Toleranzvererbungen geringere Abweichungen vom Sollmaß garantiert werden müssen. Somit kann die einteilige Ausführung einer Anschlussvorrichtung auch zu geringeren Kosten führen. Darüber hinaus wird das Risiko von Fehlern bei der Montage der Anschlussvorrichtung reduziert.

Erfindungsgemäß ist die Einweg-Anschlusseinrichtung einstückig ausgebildet. Einstückig heißt, dass die Einweg-Anschlusseinrichtung lediglich aus einem einzigen Bauteil besteht. Die bezeichneten Merkmale der Einweg-Anschlusseinrichtung sind demnach vollständig in einem einzelnen Bauteil ausgebildet, so dass keine Baugruppe oder Montage mehrerer einzelner Bauteile notwendig ist.

Der Erfindung liegt ferner die Erkenntnis zugrunde, dass die Ausbildung einer Anschlussvorrichtung aus Kunststoff vorteilhaft hinsichtlich der materialtechnischen Eigenschaften ist. Bei der Wahl eines geeigneten Kunststoffs kann die Chemikalienbeständigkeit des Kunststoffs genutzt werden, die insbesondere gegenüber chlorhaltigen Lösungen besteht. Ferner trägt eine Paarung aus Kunststoff, für die Anschlussvorrichtung, und Metall und/oder Glas, für die Kopfplatte, zur hermetischen Abdichtung der Anschlussöffnung bei, da die thermische Ausdehnung von Kunststoff höher ist als die thermische Ausdehnung von Stahl oder Glas. Wenn auch die Kopfplatte und/oder das Anschlusselement aus Kunststoff bestehen oder Kunststoff aufweisen, wird vorzugsweise für die Einweg-Anschlusseinrichtung der gleiche Kunststoff eingesetzt oder ein Kunststoff mit einer höheren thermischen Ausdehnung. Erfindungsgemäß ist die Einweg-Anschlusseinrichtung daher aus einem Kunststoff ausgebildet. Vorzugsweise werden für die Einweg-Anschlusseinrichtung Kunststoffe gewählt, die den Anforderungen United States Pharmacopeia (USP) Klasse VI entsprechen.

Die Ausbildung der Einweg-Anschlusseinrichtung aus Kunststoff ist ferner vorteilhaft durch die Möglichkeit, einen Durchgang oder mehrere oder sämtliche Durchgänge durch Hitzeschweißen und/oder Heißkleben und/oder sonstige geeignete Verfahren zu verschließen. Darüber hinaus ist die Verwendung einer Einweg-Anschlusseinrichtung aus Kunststoff bei Verwendung von Beutel-Bioreaktoren dahingehend vorteilhaft, dass das Risiko einer Beschädigung des Beutel-Bioreaktors, einer sogenannten Bagverletzung, reduziert werden kann. Weitere Vorteile ergeben sich aus Perspektive der Herstellung.

Kunststoffteile können bei entsprechender Auslegung einstückig im Spritzgussverfahren hergestellt werden. Das Verfahren ermöglicht bei Gewährleistung sehr enger Toleranzen und der Herstellung in Massenproduktion eine kostengünstige Herstellung. Ferner ermöglicht das Mehrkomponentenspritzgießen, auch Multikomponenten-Spritzguss genannt, die Anspritzung weiterer Elemente wie beispielsweise Dichtungen. Insbesondere ermöglicht das Mehrkomponentenspritzgießen die Herstellung eines einstückigen Bauteils mit Bereichen aufweisend unterschiedliche Materialeigenschaften. Ferner vorzugsweise können Elemente, wie beispielsweise ein Luer-Lock-System, direkt an die Einweg-Anschlusseinrichtung angespritzt werden oder direkt an der einstückigen Einweg-Anschlusseinrichtung vorgesehen werden.

Ein weiterer Vorteil der aus Kunststoff ausgebildeten Einweg-Anschlusseinrichtung ist die Möglichkeit eines einfachen Aufsteckens von Schläuchen und weiteren anzuschließenden Elementen an die Durchgänge bzw. an die Wandungen der Durchgänge. Darüber hinaus besteht die Möglichkeit einer bedarfsgerechten Oberflächenstrukturierung der äußeren Oberfläche der die Durchgänge umgebenden Wandungen.

In einer bevorzugten Ausführungsvariante ist die Einweg-Anschlusseinrichtung dadurch gekennzeichnet, dass die Befestigungsstruktur in Form eines Außengewindes ausgebildet ist. Der Befestigungsabschnitt mit der Befestigungsstruktur weist in dieser Ausführungsvariante vorzugsweise einen kreisrunden Querschnitt auf. Der Querschnitt ist in diesem Zusammenhang derart zu verstehen, dass es sich um einen Querschnitt orthogonal zu einer Mittelachse des Befestigungsabschnitts handelt. Vorzugsweise ist an der Innenumfangsfläche der Anschlussöffnung der Kopfplatte bzw. des Anschlusselements eine komplementäre Befestigungsstruktur in Form eins komplementären Innengewindes ausgebildet, in welches das Außengewinde eingreifen kann. In dieser Ausführungsform ist die Einweg-Anschlusseinrichtung vorzugsweise lösbar befestigbar.

Der Befestigungsabschnitt weist in dieser bevorzugten Ausführungsvariante, vorzugsweise auf einer nach außen gerichteten Oberfläche seiner Wandung, Windungen auf, die das zuvor bezeichnete Außengewinde ausbilden. Vorzugsweise weist das Außengewinde zwischen 4 und 10 Windungen und besonders bevorzugt zwischen 6 und 8 Windungen auf. Eine Windung verläuft dabei vollständig um die nach außen gerichtete Oberfläche des Befestigungsabschnitts herum, wobei der Anfang und das Ende einer Windung, hinsichtlich einer Richtung parallel zur Längsachse der Einweg-Anschlusseinrichtung, beabstandet sind. Die dadurch entstehende Spirale weist bevorzugt eine Längsachse auf, die parallel und ortsgleich zur Längsachse der Einweg-Anschlusseinrichtung verläuft.

Vorzugsweise sind im Befestigungsabschnitt und/oder am Außengewinde zusätzlich Dichtungsmittel und/oder Dichtungseinrichtungen eingebracht. Dies können beispielsweise elastische Festkörper, wie Dichtbänder, und/oder Pasten und/oder Gele und/oder Flüssigkeitsdichtungen sein. Dabei füllen die Dichtmittel und/oder Dichtungseinrichtungen vorzugsweise die Zwischenräume im Gewinde aus und erreichen eine in Abhängigkeit der gewählten Dichtmittel und/oder Dichtungseinrichtungen Dichtwirkung. Darüber hinaus vorzugsweise weist das Außengewinde eine Schraubsicherung auf, die das unbeabsichtigte Lösen der Verschraubung verhindert.

In einer besonders bevorzugten Ausführungsvariante ist die Einweg-Anschlusseinrichtung dadurch gekennzeichnet, dass die Befestigungsstruktur in Form von Rippen und/oder Vorsprüngen und/oder Schnappelementen und/oder Rastelementen und/oder Vertiefungen ausgebildet ist.

Rippen und/oder Vorsprünge und/oder Schnappelemente und/oder Rastelemente und/oder Vertiefungen sind kostengünstig erzeugbare Befestigungsstrukturen, wobei darüber hinaus das Einsetzen der Einweg-Anschlusseinrichtung in eine Anschlussöffnung mit einem geringen Zeitaufwand einher geht. Die Rippen und/oder Vorsprünge und/oder Schnappelemente und/oder Rastelemente und/oder Vertiefungen sind vorzugsweise integral mit der Einweg-Anschlusseinrichtung verbunden. Die Rippen und/oder Vorsprünge und/oder Schnappelemente und/oder Rastelemente und/oder Vertiefungen können im und/oder am Befestigungsabschnitt einzeln oder in Kombination vorgesehen werden.

In Abhängigkeit der Befestigungsstruktur ist zudem gegebenenfalls eine entsprechende Geometrie in und/oder an der Anschlussöffnung, insbesondere an deren Innenumfangsfläche, vorzusehen, damit beispielsweise ein Rastelement einrasten kann. In dieser Ausführungsform kann die Einweg-Anschlusseinrichtung vorzugsweise lösbar oder nicht lösbar ausgebildet sein. Ferner können die Rippen und/oder Vorsprünge und/oder Schnappelemente und/oder Rastelemente und/oder Vertiefungen Dichtmittel und/oder Dichteinrichtungen umfassen, um die Trennfuge zwischen Einweg-Anschlusseinrichtung und Anschlussöffnung, vorzugsweise hermetisch, abzudichten.

In einer weiteren bevorzugten Ausführungsvariante ist die Einweg-Anschlusseinrichtung dadurch gekennzeichnet, dass der Befestigungsabschnitt eine umlaufende Anschlagsfläche aufweist, welche sich von der Außenumfangsfläche in radialer Richtung nach außen erstreckt. Die umlaufende Anschlagsfläche ist vorzugsweise integral mit der Einweg-Anschlusseinrichtung verbunden. Ferner vorzugsweise beginnt die Außenumfangsfläche in Richtung der Längsachse der Einweg-Anschlusseinrichtung unmittelbar am Ende der letzten Windung des Außengewindes.

Die Einweg-Anschlusseinrichtung wird in die Anschlussöffnung eines Bioreaktors oder eines Beutel-Bioreaktors eingeführt bis ein Abschnitt der Oberfläche der Kopfplatte und die Anschlagsfläche unmittelbar aneinander anliegen. Ferner vorzugsweise liegen diese lediglich mittelbar aneinander an, da zwischen diesen ein Dichtmittel und/oder eine Dichtungseinrichtung angeordnet ist.

Eine weitere besonders bevorzugte Ausführungsvariante der Einweg-Anschlusseinrichtung sieht vor, dass der Befestigungsabschnitt einen Kraftaufnahmeabschnitt aufweist, welcher ein Mehrkantprofil umfasst und sich vorzugsweise unmittelbar an die Anschlagsfläche anschließt. Der Kraftaufnahmeabschnitt ist vorzugsweise in einem Abschnitt der Einweg-Anschlusseinrichtung angeordnet, der sich, während des ordnungsgemäßen Betriebs eines Bioreaktors mit der Einweg-Anschlusseinrichtung, außerhalb der Anschlussöffnung und außerhalb des Bioreaktors befindet. Der Kraftaufnahmeabschnitt ist ferner derart angeordnet, dass dieser von Hand und/oder mittels eines Werkzeugs zugänglich ist.

Der Kraftaufnahmeabschnitt dient zur Einbringung einer Kraft, vorzugsweise einer Kraft in tangentialer Richtung, um die Einweg-Anschlusseinrichtung in der Anschlussöffnung eines Bioreaktors zu befestigen und/oder um die Befestigung der Einweg-Anschlusseinrichtung zu lösen. Die Krafteinwirkung auf den Kraftaufnahmeabschnitt wird erfindungsgemäß derart verbessert, dass ein Mehrkantprofil an dem Kraftaufnahmeabschnitt vorgesehen ist. Das Mehrkantprofil am Befestigungsabschnitt der Einweg-Anschlusseinrichtung hat die gleiche Funktion und eine geometrisch analoge Ausprägung wie die Mehrkantprofile bei Schraubenköpfen und/oder Muttern, wobei bei diesen vorzugsweise Sechskant- oder Vierkantköpfe verwendet werden. Das Mehrkantprofil am Befestigungsabschnitt der Einweg-Anschlusseinrichtung ist vorzugsweise als Zweikant-, ferner vorzugsweise als Vierkant-, ferner vorzugsweise als Sechskant-, ferner vorzugsweise als Achtkant-, ferner vorzugsweise als Zehnkant-, ferner vorzugsweise als Zwölfkant- und ferner vorzugsweise als Vierzehnkantprofil ausgebildet. Die Höhe des Mehrkantprofils in Richtung der Längsachse der Einweg-Anschlusseinrichtung weist vorzugsweise eine Abmessung zwischen 2 mm und 10 mm und besonders bevorzugt zwischen 4 mm und 8 mm auf.

In einer weiteren besonders bevorzugten Ausführungsvariante ist die Einweg-Anschlusseinrichtung, dadurch gekennzeichnet, dass ein Durchgang, mehrere oder sämtliche Durchgänge zumindest abschnittsweise als Rohrsegment ausgebildet sind, wobei vorzugsweise sich ein Rohrsegment, mehrere oder sämtliche Rohrsegmente parallel zu einer Mittelachse der Außenumfangsfläche in eine erste Richtung von der Außenumfangsfläche und/oder in eine zweite Richtung von der Außenumfangsfläche erstrecken.

Ein Durchgang, mehrere Durchgänge oder sämtliche Durchgänge weisen in dieser Ausführungsvariante zumindest abschnittsweise einen kreisrunden Querschnitt auf. Das zumindest abschnittsweise Aufweisen eines Rohrsegments heißt, dass ein Durchgang, mehrere Durchgänge oder sämtliche Durchgänge entweder vollständig über die gesamte axiale Länge des jeweiligen Durchgangs einen kreisrunden Querschnitt oder diesen lediglich in gewissen Abschnitten entlang der axialen Länge des jeweiligen Durchgangs aufweisen. Abschnitte, die kein Rohrsegment aufweisen, können unterschiedlich geometrisch geformte Querschnitte aufweisen. Insbesondere können die Querschnitte des und/oder der Durchgänge schlitzförmig, dreieckig, viereckig oder mehreckig sein. Ferner bevorzugt kann der Querschnitt auch eine ovale Form annehmen. Die Durchtrittsrichtung des Rohrsegments oder der Rohrsegmente bzw. des Durchgangs oder der Durchgänge ist in dieser Ausführungsvariante parallel zu der Mittelachse der Außenumfangsfläche.

Vorzugsweise ist ein Rohrsegment oder sind mehrere oder sämtliche Rohrsegmente derart, beispielsweise dünnwandig, ausgebildet, dass diese mittels eines Schneidwerkzeugs kürzbar sind. Schneidwerkzeug kann z.B. eine Zange, Schere oder ein ähnliches, zum Kürzen und/oder kappen der Rohrsegmente geeignetes Laborinstrument sein.

In einer weiteren bevorzugten Ausführungsvariante ist die Einweg-Anschlusseinrichtung dadurch gekennzeichnet, dass ein Rohrsegment, mehrere oder sämtliche Rohrsegmente eine oder mehrere Sollbruchstellen aufweisen. Sollbruchstellen sollen im Allgemeinen im Überlastfall gezielt und vorhersagbar versagen, um hierdurch Schaden an einem Gesamtsystem zu vermeiden. Dieses Prinzip wird bei der Einweg-Anschlusseinrichtung genutzt, um an den Rohrsegmenten vorbestimmte Stellen zu definieren, an denen die Rohrsegmente gekappt und/oder auf vorbestimmte Längen gekürzt werden können, vorzugsweise ohne Werkzeuge, sondern lediglich durch entsprechende Verformung, wie biegen und/oder brechen, per Hand durch einen Benutzer.

Die Sollbruchstellen sind daher durch konstruktive, mechanische bzw. physikalische Maßnahmen dahingehend ausgelegt, dass bei einer geeigneten Krafteinbringung das Rohrsegment an einer Sollbruchstelle getrennt wird. Die Kürzung der Rohrsegmente kann somit ohne großen Aufwand durchgeführt werden. Ferner ist durch den geringen benötigten Kraftaufwand das Risiko einer Beschädigung der Einweg-Anschlusseinrichtung, beispielsweise der Integrität, reduziert. Vorzugsweise sind die Sollbruchstellen als Einkerbungen ausgebildet.

Eine weitere bevorzugte Ausführungsvariante der Einweg-Anschlusseinrichtung sieht vor, dass ein Rohrsegment, mehrere oder sämtliche Rohrsegmente unter Wärmeeinwirkung zumindest derart verformbar sind, dass der jeweilige Durchgang durch Formveränderung fluiddicht verschließbar ist. Die Wärmeeinwirkung erfolgt vorzugsweise durch Hitzeschweißen, Heißkleben oder dergleichen. Die Verformbarkeit der Rohrsegmente wird insbesondere durch die Wahl des Materials der Rohrsegmente bestimmt. Hier ist vorzugsweise ein thermoplastischer Kunststoff vorzusehen, so dass ein Rohrsegment oder mehrere oder sämtliche Rohrsegmente aus einem aufschmelzbaren Kunststoff ausgebildet sind. Durch die Wärmeeinwirkung und die anschließende Verformung des Rohrsegments oder der Rohrsegmente wird der Durchgang fluiddicht verschlossen. Fluiddicht verschlossen heißt, dass durch den fluiddicht verschlossenen Durchgang kein Fluid durchströmen oder anderweitig von einem ersten Ende des Durchgangs zu einem zweiten Ende des Durchgangs gelangen kann.

Diese Ausführungsvariante erhöht die Praktikabilität der Einweg-Anschlusseinrichtung, da diese universeller einsetzbar ist. Bei Bedarf einer Anzahl von Durchgängen, die geringer ist als die Anzahl der zur Verfügung stehenden Durchgänge an der Einweg-Anschlusseinrichtung, besteht für den Anwender die Möglichkeit, einen oder mehrere Durchgänge zu verschließen. Infolgedessen sind lediglich so viele Durchgänge vorhanden, wie auch benötigt werden. Dies vereinfacht für den Anwender die Anwendung, da er keine große Anzahl an unterschiedlichen Anschlusseinrichtungen vorhalten muss und das Verwechslungsrisiko zusätzlich reduziert wird. Ferner können durch Skaleneffekte die Einkaufskosten reduziert werden.

In einer weiteren besonders bevorzugten Ausführungsvariante ist die Einweg-Anschlusseinrichtung dadurch gekennzeichnet, dass ein Rohrsegment, mehrere oder sämtliche Rohrsegmente an zumindest einem Ende mit einer Schlauchsicherungseinrichtung abschließen.

Für eine erfolgreiche Kultivierung von Mikroorganismen und/oder Zellkulturen ist es wichtig, dass die Einflussfaktoren im Bioreaktor, beispielsweise Temperatur, Nährstoffversorgung, PH-Wert, Sauerstoffgehalt, Homogenisierung und/oder Schaumbildung, den vom Anwender definierten Wert oder definierten Wertebereich aufweisen.

Eine sichere Zu- und/oder Ableitung von Medien ist wichtig, um die bezeichneten Einflussfaktoren im Bioreaktor im definierten Wertebereich zu halten. Die Zu- und Ableitungen werden durch die Durchgänge in der Einweg-Anschlusseinrichtung sowie durch an diese angeschlossene Schläuche gewährleistet. Eine sichere Zu- und Ableitung von Medien wird unter anderem durch Schlauchsicherungseinrichtungen gewährleistet, die ein unzulässiges oder unbeabsichtigtes Entfernen der Schläuche, welche mit den Durchgängen in der Einweg-Anschlusseinrichtung verbunden sind, vermeiden. Vorzugsweise ist die Schlauchsicherungseinrichtung als Luer-Lock-System ausgebildet.

In einer weiteren Ausführungsvariante ist mindestens ein Rohrsegment aus Kunststoff gebildet und weist an einem zur Einbringung in den Bioreaktor vorgesehenen Ende ein Filterelement auf.

Das zur Einbringung in den Bioreaktor vorgesehene Ende wird auch als inneres Ende bezeichnet. Über die Verwendung eines Filterelementes oder Filters kann zum einen sichergestellt werden, dass keine Zellen aus der Kulturbrühe bei einer Verwendung des jeweiligen Rohrsegmentes für eine Medienableitung den Bioreaktor verlassen, da diese vom Filter aufgehalten werden. Die Verwendung eines Filters ist aber auch vorteilhaft in Rohrsegmenten für die Medien- oder Gastzufuhr, über die poröse Struktur des Filters wird das zugeführte Medium oder das Gas feinverteilt in die Kulturbrühe geleitet. Es ist daher insbesondere vorteilhaft, wenn das Rohrsegment, das das Filterelement aufweist, ausreichend lang ausgebildet ist, so dass bei Einsatz im Bioreaktor das Ende des Rohrsegmentes und insbesondere auch das Filterelement in der Kulturbrühe angeordnet ist.

Das Filterelement weist je nach Anwendungsfall bevorzugt eine Porengröße in einem Bereich von 0,1 µm - 500 µm, insbesondere in einem Bereich von 0,1 - 0,2 µm, von 0,1 bis 1 µm, von 0,5 - 5 µm, von 5 - 50 µm, von 5 - 250 µm, von 20- 40µm, von 20- 100 µm, von 20- 250 µm oder von 5 - 500 µm auf.

Die Porengröße kann vorzugsweise als nominale Porengröße, also als Maximum der Porengrößenverteilung, verstanden werden. In weiteren Ausführungsformen kann die Porengröße so verstanden werden, dass das Filterelement ausschließlich Poren aufweist, deren Porengröße innerhalb der genannten Wertebereiche liegt. Die Bestimmung der Porengrößen kann durch Quecksilberintrusion, wie in der DIN66133 vom Juni 1993 beschrieben, bestimmt werden. Die DIN66133 kann über den Beuth-Verlag, Am DIN-Platz, Burggrafenstraße 6, 10787 Berlin bezogen werden.

Das Filterelement ragt in einer Ausführungsvariante über das Ende des Rohrsegmentes hinaus, so dass es großflächig vom Medium umgeben sein kann. In einer alternativen Ausführungsform schließt das Filterelement bündig mit dem Ende des Rohrsegmentes ab oder ist in Bezug auf dieses leicht zurückversetzt. In dieser Ausführungsform trifft das Medium vorwiegend auf die Stirnfläche des Filterelementes.

Das Filterelement kann eine Membran enthalten und/oder eine poröse Struktur aufweisen. Insbesondere kann das Filterelement einen Glasfilter, eine poröse Keramik, insbesondere eine Schaumkeramik, einen Kunststofffilter oder eine Filterkassette umfassen oder daraus bestehen. Vorzugsweise umfasst das Filterelement Borosilikat und/oder Aluminiumoxid und/oder Zirkonoxid und/oder Polycarbonat und/oder Polypropylen und/oder Polyethylen. Ein Glasfilter kann vorzugsweise aus Borosilikat bestehen oder Borosilikat umfassen. Eine poröse Keramik kann vorzugsweise aus Aluminiumoxid und/oder Zirkonoxid bestehen oder Aluminiumoxid und/oder Zirkonoxid umfassen. Ein Kunststofffilter kann vorzugsweise aus Polycarbonat und/oder Polypropylen und/oder Polyethylen bestehen oder Polycarbonat und/oder Polypropylen und/oder Polyethylen umfassen. Eine Filterkassette kann vorzugsweise gefüllt sein, beispielsweise mit Kieselgur und/oder Fasern und/oder anderen Materialien.

Vorzugsweise kann das Filterelement modifiziert und/oder nachbehandelt sein, insbesondere beschichtet und/oder aktiviert, insbesondere derart, dass eine Oberfläche des Filterelementes hydrophob oder hydrophil ist.

Vorzugsweise ist der Kunststoff des mindestens einen Rohrsegments ein thermoplastischer Kunststoff.

Bevorzugt werden für das Filterelement und/oder das mindestens eine Rohrsegment Materialien verwendet, die unter USP Class VI fallen.

Vorzugsweise sind das Rohrsegment und das Filterelement integral über hybriden Spritzguss ausgebildet, bei dem zunächst das Filterelement spritzgegossen oder durch ein anderes Formgebungsverfahren hergestellt und/oder gesintert wird und anschließend das Rohrsegment, in Teilen um das vorgefertigte, gegebenenfalls bereits gesinterte, Filterelement spritzgegossen wird. Das Filterelement kann mit dem Rohrsegment beispielsweise verklebt, verrastet, verschweißt oder über einen Presssitz verbunden sein.

In einer weiteren bevorzugten Ausführungsvariante ist die Einweg-Anschlusseinrichtung gekennzeichnet durch einen Sensor oder mehrere Sensoren und/oder eine Elektrode oder mehrere Elektroden, wobei vorzugsweise in einem Durchgang, mehreren oder sämtlichen Durchgängen mindestens ein Sensor und/oder mindestens eine Elektrode angeordnet ist. Ein Sensor kann eine oder mehrere Elektrode umfassen oder als Elektrode ausgebildet sein. Beim Vorsehen mehrere Elektroden sind diese vorzugsweise voneinander beabstandet und/oder voneinander elektrisch isoliert. Die Elektrode(n) ist bzw. sind vorzugsweise als Stabelektrode(n) oder als im Wesentlichen stabförmige(r) Elektrodenträger mit daran befestigter/n Elektrode(n) ausgebildet.

In einer bevorzugten Ausgestaltung weist die Einweg-Anschlusseinrichtung mindestens zwei beabstandete, voneinander elektrisch isolierte Elektroden auf, die vorzugsweise jeweils in einem Durchgang angeordnet sind. Alternativ können zwei oder mehrere Elektroden und/oder Sensoren auch gemeinsam in einem Durchgang mit entsprechendem Durchmesser angeordnet sein. Wenn beispielsweise die in einem Durchgang angeordneten Elektroden und/oder Sensoren in unterschiedlicher Höhe im Durchgang enden und der Durchgang als Tauchrohr dimensioniert ist, kann auch innerhalb des Durchgangs beispielsweise der Füllstand wie im folgenden beschrieben detektiert werden. Durch einen möglichen Kapillareffekt hervorgerufene Abweichungen können durch entsprechende Kalibrierung und/oder Dimensionierung des Durchgangs behoben oder reduziert werden.

Beispielsweise kann ein Sensor als Füllstandsmesser ausgebildet sein, der die Höhe des im Reaktor befindlichen Mediums misst, wobei die Höhe vorzugsweise in einer vertikalen Richtung gemessen wird. Der Füllstandsmesser der Einweg-Anschlusseinrichtung kann bevorzugt mit mindestens einer Elektrode, vorzugsweise zwei oder mehr Elektroden, ausgebildet sein, wobei die Messung über dem Medium oder im Medium erfolgt. Wenn der Füllstandsmesser der Einweg-Anschlusseinrichtung nur eine Elektrode aufweist, ist vorzugsweise mindestens eine weitere Elektrode an anderer Stelle an oder in dem Bioreaktor angeordnet, damit insbesondere ab dem Erreichen eines bestimmten Füllstands eine Spannung anliegen kann. Insbesondere bei Stahltank-Bioreaktoren oder Bioreaktoren aus anderen leitfähigen Materialien kann beispielsweise die Reaktorwand eine solche weitere Elektrode bilden. Die Bestimmung der Höhe erfolgt ferner bevorzugt durch die Messung einer Spannung zwischen mindestens zwei Elektroden.

In einer weiteren Ausführungsform kann mit dem mindestens einen Sensor und/oder der mindestens einen Elektrode auch eine Schaumbildung messbar sein. In diesem Fall wird vorzugsweise die elektrische Leitfähigkeit zwischen den zwei Elektroden gemessen, wobei wie zuvor beschrieben die erste Elektrode vorzugsweise an der Einweg-Anschlusseinrichtung angeordnet ist und die zweite Elektrode am Bioreaktor angeordnet sein kann (beispielsweise als Wandung eines Stahlbioreaktors) oder ebenfalls an der Einweg-Anschlusseinrichtung angeordnet sein kann. Schaum weist im Vergleich zum Medium eine andere, insbesondere geringere, elektrische Leitfähigkeit auf. Über die Messung der elektrischen Leitfähigkeit und einen Vergleich mit Referenzwerten für Schaum und/oder Medium kann somit eine Schaumbildung im Bioreaktor festgestellt werden.

In einer weiteren Ausgestaltungsform weist eine erfindungsgemäße Einweg-Anschlusseinrichtung einen Sensor mit mindestens drei Elektroden auf, von denen eine erste Elektrode als Füllstandsmesser für das Medium, eine zweite Elektrode für die Messung einer Schaumbildung und eine dritte Elektrode als gemeinsame Gegenelektrode ausgebildet und angeordnet ist.

Der mindestens eine Sensor und/oder die mindestens eine Elektrode kann entweder in einen Durchgang oder mehrere Durchgänge der einstückigen Einweg-Anschlusseinrichtung eingeführt werden oder integral mit der Einweg-Anschlusseinrichtung verbunden sein. Letztgenannte Variante erfordert in der Regel einen Spritzgießprozess, in dem der mindestens eine Sensor und/oder die mindestens eine Elektrode als Einlegeteil umspritzt wird bzw. werden. Das Umspritzen bietet den Vorteil eines integralen Bauteils, das nicht montiert werden muss sowie einer hohen Dichtigkeit zwischen der Elektrode und dem umgebenden Material der Einweg-Anschlusseinrichtung. Ferner ist die axiale Position des mindestens einen Sensors und/oder der mindestens einen Elektrode in der Einweg-Anschlusseinrichtung definiert, so dass hier bei der Montage oder bei der Prozesseinrichtung ein geringeres Fehlerrisiko besteht.

In einer weiteren besonders bevorzugten Ausführungsvariante ist die Einweg-Anschlusseinrichtung gekennzeichnet durch eine umlaufende Dichtung zum fluiddichten Abdichten gegenüber dem Bioreaktor. Die umlaufende Dichtung ist vorzugsweise an einem Abschnitt der umlaufenden Anschlagsfläche angeordnet, wobei dieser Abschnitt im montierten Zustand der Oberfläche der Kopfplatte des Bioreaktors und/oder der Oberfläche des Beutel-Bioreaktors zugeneigt ist. Durch eine Abdichtung dieser Trennfuge wird die hermetische Abdichtung des Innenraums des Bioreaktors verbessert. Als Dichtmittel sind konventionelle Dichtringe aus einem elastischen Material und/oder abdichtende Pasten, Gele und/oder andere Dichtmaterialien möglich. Ferner vorzugsweise ist die umlaufende Dichtung integral mit der Einweg-Anschlusseinrichtung verbunden. Diese integrale Bauweise wird beispielsweise durch das Anspritzen einer Dichtung im Herstellungsprozess ermöglicht, wobei hier insbesondere ein Mehrkomponentenspritzgießprozess eingesetzt wird.

Gemäß einem weiteren Aspekt der Erfindung wird die eingangs genannte Aufgabe gelöst durch eine Kopfplatte für einen Bioreaktor, mit einer Anschlussöffnung, wobei die Kopfplatte dazu ausgebildet ist, an einem Behälter des Bioreaktors befestigt zu werden, und einer in die Anschlussöffnung eingesetzten oder einsetzbaren Anschlusseinrichtung, dadurch gekennzeichnet, dass die Anschlusseinrichtung nach mindestens einer der zuvor beschriebenen Ausführungsformen der Einweg-Anschlusseinrichtung ausgebildet ist.

Der Bioreaktor weist in der Regel eine vollständig geschlossene Umwandung auf, wobei eine Seite von einer Kopfplatte abgeschlossen ist. Die Kopfplatte hat die Funktion, die vollständige Umwandung des Bioreaktors und/oder die Zu- und/oder Abführung von Medien sowie von Instrumenten zu gewährleisten. Die Kopfplatte ist vorzugsweise mit einer Umwandung des Bioreaktors, vorzugsweise ein Behälter mit einer Behälteröffnung, verbunden. Ferner vorzugsweise verschließt die Kopfplatte die teilweise offene Umwandung des Behälters und/oder die Behälteröffnung, um den Behälter hermetisch abzudichten. Die Verbindung kann insbesondere in Abhängigkeit des Materials der Kopfplatte und des Materials des Behälters stoffschlüssig und/oder kraftschlüssig und/oder formschlüssig ausgebildet sein. Die Kopfplatte kann aus metallischen und/oder keramischen Materialien und/oder Kunststoff bestehen oder diese umfassen.

Die Kopfplatte weist darüber hinaus mindestens eine Anschlussöffnung auf, die der Aufnahme bzw. der Befestigung der Einweg-Anschlusseinrichtung dient. Die Anschlussöffnung weist ferner vorzugsweise eine Struktur auf, die zur Aufnahme der Befestigungsstruktur der Einweg-Anschlusseinrichtung ausgebildet ist. Bei einer Ausführung der Einweg-Anschlusseinrichtung mit einem Außengewinde, weist die Anschlussöffnung vorzugsweise ein Innengewinde auf.

Die Kopfplatte weist darüber hinaus eine flächige Ausdehnung auf, wobei die flächige Ausdehnung im Betriebszustand, das heißt montiert an einem Behälter des Bioreaktors und aufgestellt auf eine im Wesentliche horizontale Arbeitsfläche, vorzugsweise eine im Wesentlichen horizontale Erstreckung aufweist. Eine Dicke, gemessen horizontal zur flächigen Erstreckung, weist im Vergleich zur flächigen Erstreckung eine geringe Abmessung auf. Die Kopfplatte kann vorzugsweise eine überwiegend ebene Erstreckung aufweisen. Alternativ vorzugsweise kann die Kopfplatte auch eine gewölbte Erstreckung aufweisen. Ferner kann die Kopfplatte auch Verstärkungselemente aufweisen, beispielsweise Verippungen.

Die Einweg-Anschlusseinrichtungen werden in der Regel in oder an die Anschlussöffnungen der Kopfplatte angebracht. Vorzugsweise weisen diese Anschlussöffnungen, insbesondere im Betrieb des Bioreaktors bei Aufstellung auf einer im Wesentlichen horizontalen Arbeitsfläche, eine im Wesentlichen vertikale Durchtrittsrichtung auf. Die Anschlussöffnungen weisen einen Querschnitt orthogonal zu ihrer Mittelachse auf. Dieser Querschnitt kann vorzugsweise kreisrund, und/oder schlitzförmig, und/oder dreieckig, und/oder viereckig, und/oder mehreckig sein. Ferner bevorzugt kann der Querschnitt auch eine ovale Form annehmen.

Gemäß einem weiteren Aspekt der Erfindung wird die eingangs genannte Aufgabe gelöst durch einen Bioreaktor zur Kultivierung von Mikroorganismen und/oder Zellkulturen, mit einem Behälter und einer Kopfplatte zum Verschließen des Behälters, dadurch gekennzeichnet, dass die Kopfplatte gemäß dem zuvor beschriebenen Aspekt ausgebildet ist.

Durch einen solchen Bioreaktor mit einer erfindungsgemäßen Kopfplatte und umfassend eine erfindungsgemäße Einweg-Anschlusseinrichtung können verschiedene Nachteile bestehender Ausführungen von Bioreaktoren verringert oder behoben werden. Durch die Einwegausführung, die Einstückigkeit und Chemikalienbeständigkeit der Einweg-Anschlusseinrichtung wird insbesondere das Risiko einer Kontamination des Bioreaktors verringert. Ferner erhöhen die Einstückigkeit und die Möglichkeit der sicheren Anbringung von Schläuchen die Betriebssicherheit des Bioreaktors.

Es ist dabei insbesondere vorteilhaft, wenn die Anschlusseinrichtung mindestens ein Rohrsegment umfasst, dessen eines im Bioreaktor angeordnetes Ende sich unterhalb einer Mindestfüllhöhe für Kulturmedium des Bioreaktors befindet. Das Rohrsegment umfasst vorzugsweise das Filterelement. Daher kann es auch ausreichen, wenn sich nur das Filterelement bzw. dessen - im Betriebszustand unteres - Ende unterhalb der Mindestfüllhöhe befindet. Damit ist sichergestellt, dass das Rohrsegment in das Kulturmedium ragt, unabhängig von der tatsächlichen Füllhöhe im Bioreaktor. Wichtig ist bei dieser Ausführungsform, dass das Rohrsegment derart ausgebildet ist, dass es in das Kulturmedium ragt. So kann über das Rohrsegment eine Begasung des Kulturmediums erfolgen, in dem Luft oder auch andere Gase direkt in das Kulturmedium eingeleitet werden können. Alternativ kann das Rohrsegment genutzt werden, um Kulturmedium oder Produkte der im Kulturmedium enthaltenen Zellen abzuziehen. Damit kann sowohl ein Medienaustausch als auch eine Produktgewinnung realisiert werden. Insbesondere ist es vorteilhaft, wenn das jeweilige Rohrsegment abwechselnd zur Begasung und zum Abzug dient, da dann ein Rückspülen am Rohrsegment stattfindet und eine Verunreinigung des Rohrsegmentes beispielsweise über verbleibende Reste von Medium vermieden wird. Es ist dabei insbesondere vorteilhaft, wenn das Rohrsegment wie bereits beschrieben ein Filterelement aufweist. Dieses kann je nach Anwendung für eine feinblasige Begasung sorgen und unerwünschten Abzug, beispielsweise der Zellen im Kulturmedium vermeiden.

Gemäß einem weiteren Aspekt der Erfindung wird die eingangs genannte Aufgabe gelöst durch ein Anschlusselement für einen Beutel-Bioreaktor, mit einer Anschlussöffnung, wobei das Anschlusselement dazu ausgebildet ist, mit einem Beutelbehälter verbunden zu werden und/oder einstückig mit diesem ausgebildet zu werden, und einer in die Anschlussöffnung eingesetzten oder einsetzbaren Anschlusseinrichtung, dadurch gekennzeichnet, dass die Anschlusseinrichtung nach mindestens einer der zuvor beschriebenen Ausführungsformen der Einweg-Anschlusseinrichtung ausgebildet ist.

Gemäß einem weiteren Aspekt der Erfindung wird die eingangs genannte Aufgabe gelöst durch einen Beutel-Bioreaktor zur Kultivierung von Mikroorganismen und/oder Zellkulturen, mit einem verformbaren Beutelbehälter mit einem Anschlusselement, dadurch gekennzeichnet, dass das Anschlusselement gemäß dem zuvor beschriebenen Aspekt ausgebildet ist.

Auch hier ist es vorteilhaft, wenn die Anschlusseinrichtung des Anschlusselementes mindestens ein Rohrsegment umfasst, dessen eines im Beutel-Bioreaktor angeordnetes Ende sich unterhalb einer Mindestfüllhöhe für Kulturmedium des Beutel-Bioreaktors befindet.

Für weitere Vorteile, Ausführungsvarianten und Ausführungsdetails dieser weiteren Aspekte und ihrer möglichen Fortbildungen wird auch auf die zuvor erfolgte Beschreibung zu den entsprechenden Merkmalen und Fortbildungen der Einweg-Anschlusseinrichtung verwiesen.

Bevorzugte Ausführungsformen der Erfindung werden beispielhaft anhand der beiliegenden Figuren erläutert. Es zeigen:
- Figur 1:: eine dreidimensionale Darstellung einer ersten beispielhaften Einweg-Anschlusseinrichtung,
- Figur 2:: eine Seitenansicht der Einweg-Anschlusseinrichtung gemäß Figur 1,
- Figur 3a:: eine längsgeschnittene Schnittdarstellung der Einweg-Anschlusseinrichtung gemäß Figur 1,
- Figur 3b:: eine längsgeschnittene Schnittdarstellung einer möglichen Variante der Einweg-Anschlusseinrichtung gemäß Figur 1,
- Figur 4:: eine dreidimensionale Darstellung einer zweiten beispielhaften Einweg-Anschlusseinrichtung,
- Figur 5:: eine längsgeschnittene Schnittdarstellung einer Einweg-Anschlusseinrichtung gemäß Figur 4,
- Figur 6:: eine Draufsicht auf die Einweg-Anschlusseinrichtung gemäß Figur 4,
- Figur 7a: eine schematische Darstellung eines Bioreaktors mit Kopfplatte, und
- Figur 7b: eine schematische Darstellung eines Beutel-Bioreaktors mit Anschlusselement,
- Figur 8: eine längsgeschnittene Schnittdarstellung einer weiteren beispielhaften Ausgestaltung einer Einweg-Anschlusseinrichtung angeordnet in einer Kopfplatte eines Bioreaktors.

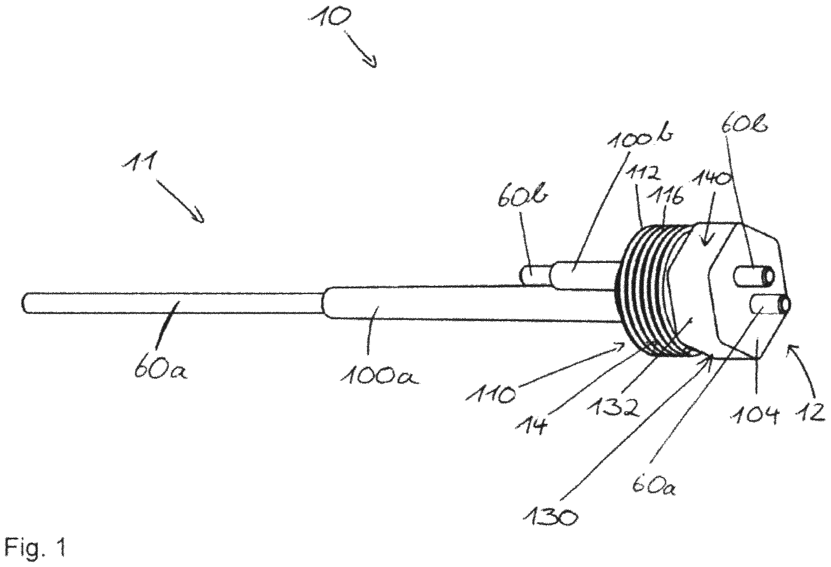

Figur 1 zeigt eine dreidimensionale Darstellung einer ersten beispielhaften Ausführungsform einer Einweg-Anschlusseinrichtung 10 mit einem ersten als Füllstandsmesser ausgebildeten Sensor 60a und einem zweiten als Füllstandsmesser ausgebildeten Sensor 60b. Die Einweg-Anschlusseinrichtung 10 umfasst ein äußeres Ende 12 an dem eine Begrenzungsschreibe 104 angeordnet ist. Die Begrenzungsscheibe 104 deckt eine Stirnseite eines als Hülse ausgebildeten Kraftaufnahmeabschnitts 130 ab, wobei die von der Begrenzungsscheibe 104 abgedeckte Stirnseite dem äußeren Ende 12 zugewandt ist. Der als Hülse ausgebildete Kraftaufnahmeabschnitt 130 weißt eine äußere Umfangsfläche auf. Diese äußere Umfangsfläche des Kraftaufnahmeabschnitts 130 weist ein Mehrkantprofil 140 auf, das hier als Sechskantprofil ausgebildet ist. Das Mehrkantprofil 140 besteht aus sechs ebenen, gleich großen Flächen 142, die auf der äußeren Umfangsfläche des Kraftaufnahmeabschnitts 130 gleichmäßig verteilt sind. Die einzelnen Flächen 142 sind derart angeordnet, dass deren jeweilige Flächennormale durch die Mittelachse einer Hülse 14 verläuft und jeweils zwei Kanten einer einzelnen Fläche wiederum an je eine Kante von zwei weiteren Flächen angrenzen.

In Längsrichtung, in Richtung des inneren Endes 11, ist an den Kraftaufnahmeabschnitt 130 ein Befestigungsabschnitt 110 angeordnet, der die gleiche Mittelachse aufweist wie der Kraftaufnahmeabschnitt 130. Der Befestigungsabschnitt 110 ist ebenfalls als Hülse mit einem konstanten Durchmesser ausgebildet, wobei der Durchmesser des Befestigungsabschnitts 110 kleiner ist als der Durchmesser des Kraftaufnahmeabschnitts 130. Dadurch entsteht am Übergang vom Kraftaufnahmeabschnitt 130 zum Befestigungsabschnitt 110 eine Stufe bzw. ein Absatz. Dieser Absatz dient als Anschlagsfläche 120, so dass die Einweg-Anschlusseinrichtung 10 bis zu diesem Übergang in eine Anschlussöffnung eines Bioreaktors eingeführt wird. Ferner weist der Befestigungsabschnitt 110 eine Außenumfangsfläche 112 auf, auf der ein Außengewinde 116 angebracht ist.

Aus der Hülse, die den Befestigungsabschnitt 110 ausbildet, treten ein erster innerer Rohrabschnitt 100a und ein zweiter innerer Rohrabschnitt 100b mit jeweils einer Durchtrittsrichtung in Richtung des inneren Endes 11 heraus. Die Auskraglänge, bezogen auf den Austritt aus dem Befestigungsabschnitt 110, der zwei inneren Rohrabschnitte 100a, 100b ist unterschiedlich, wobei der erste innere Rohrabschnitt 100a etwa die dreifache Auskraglänge aufweist als der zweite innere Rohrabschnitt 100b. Ferner zeigt Figur 1 einen ersten Füllstandsmesser 60a und einen zweiten Füllstandsmesser 60b, die hier als Stabelektroden ausgebildet sind. Die zwei Füllstandsmesser 60a, 60b haben im Wesentlichen die Aufgabe, die vertikale Höhe eines im Bioreaktor befindlichen Mediums zu messen und diesen Wert in einer geeigneten Form an eine weitere Einrichtung weiterzugeben. Dabei ist der eine Füllstandsmesser im Wesentlichen dafür ausgebildet, den oberen Füllstand zu detektieren und der jeweils andere Füllstandsmesser ist im Wesentlichen dafür ausgebildet, den unteren Füllstand zu detektieren.

Die zwei Füllstandsmesser 60a, 60b können auch dazu dienen, eine Schaumbildung zu messen. In diesem Fall wird vorzugsweise die elektrische Leitfähigkeit zwischen den zwei Elektroden gemessen, wobei. Schaum sich durch eine im Vergleich zum Medium geringere elektrische Leitfähigkeit auszeichnet und so eine Schaumbildung im Bioreaktor detektiert werden kann.

Der Füllstandsmesser 60a ist mit einem Teil seiner longitudinalen Erstreckung in dem Durchgang des ersten inneren Rohrabschnitts 100a angeordnet. Der Füllstandsmesser 60a kann in den Durchgang entweder eingeführt oder bereits im Produktionsprozess der Einweg-Anschlusseinrichtung 10 umspritzt werden. Die Anordnung und Einbringung des ersten Füllstandsmesser 60a gilt analog für die Anordnung und Einbringung des zweiten Füllstandsmesser 60b in den Durchgang des zweiten inneren Rohrabschnitts 100b. Vorzugsweise reichen die Durchgänge der inneren Rohrsegmente 100a, 100b bis zum äußeren Ende 12, so dass die Begrenzungsscheibe 104 zwei Durchgangsöffnungen aufweist. Ferner vorzugsweise treten die Füllstandsmesser 60a, 60b jeweils mit einem Ende aus der Einweg-Anschlusseinrichtung 10 heraus, so dass beispielsweise die Möglichkeit zum Anschluss der Füllstandsmesser 60a, 60b an weitere Vorrichtungen besteht.

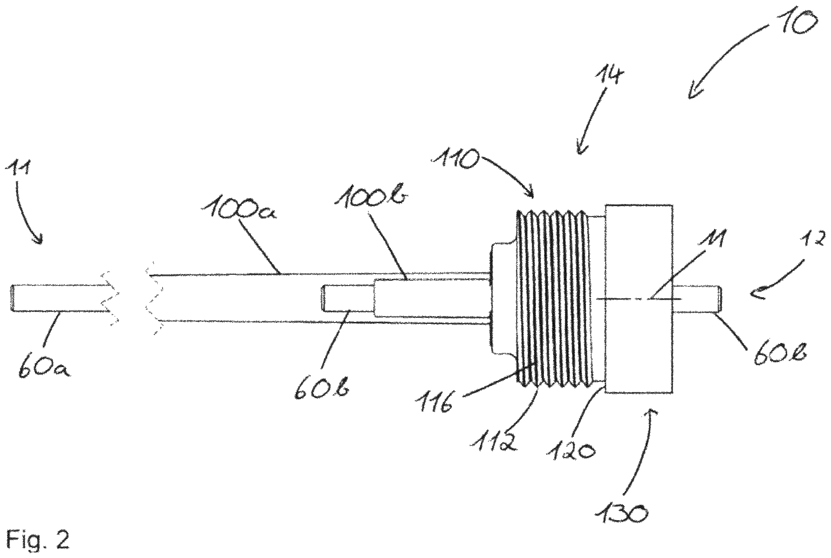

Figur 2 zeigt eine Seitenansicht der Einweg-Anschlusseinrichtung gemäß Figur 1 mit einem ersten Füllstandsmesser 60a und einem zweiten Füllstandsmesser 60b. Am äußeren Ende 12 der Einweg-Anschlusseinrichtung 10 tritt jeweils ein Ende eines ersten Füllstandsmessers 60a und eines zweiten Füllstandsmessers 60b aus der Einweg-Anschlusseinrichtung 10 aus. Das Ende des ersten Füllstandsmessers 60a am äußeren Ende 12 ist in der Figur 2 von dem Ende des zweiten Füllstandsmessers 60b verdeckt, so dass erstgenanntes nicht abgebildet ist. Die Enden der Füllstandsmesser 60a, 60b ragen demnach am äußeren Ende 12 aus einer Hülse 14 heraus, die einen Kraftaufnahmeabschnitt 130, eine Anschlagsfläche 120 und einen Befestigungsabschnitt 110 umfasst. Ferner weist die Hülse eine Mittelachse M auf, die ebenfalls die Mittelachse des Kraftaufnahmeabschnitts 130 und des Befestigungsabschnitts 110 ist.

Der Kraftaufnahmeabschnitt 130 ist als Hülse ausgebildet, wobei an den Kraftaufnahmeabschnitt 130 in Richtung des inneren Endes 11 der Befestigungsabschnitt 110 angeordnet ist. Der Befestigungsabschnitt ist ebenfalls als Hülse ausgebildet, wobei der Durchmesser des Befestigungsabschnitts 110 kleiner ist als der Durchmesser des Kraftaufnahmeabschnitts 130. Durch diese Durchmesserdifferenz entsteht am Übergang vom Befestigungsabschnitts 110 zum Kraftaufnahmeabschnitt 130 ein Absatz, durch den eine Fläche ausgebildet wird, deren Flächennormale orthogonal zur Mittelachse M ausgerichtet ist. Diese Fläche dient als Anschlagsfläche 120, so dass die Einweg-Anschlusseinrichtung 10 beim Einsetzen in eine Anschlussöffnung eines Bioreaktors bis zu dieser Anschlagsfläche 120 in die Anschlussöffnung eingeführt wird. Der Kraftaufnahmeabschnitt 130 bleibt vorzugsweise vollständig außerhalb der Anschlussöffnung.

Der Befestigungsabschnitt 110 weist eine Außenumfangsfläche 112 auf, an der eine integral gestaltete Befestigungsstruktur 114 angeordnet ist. Die Befestigungsstruktur 114 ist in diesem Ausführungsbeispiel als Außengewinde 116 ausgebildet, das eine Anzahl an Windungen aufweist. Die zwei inneren Rohrsegmente 100a, 100b sind parallel zur Mittelachse M angeordnet. Ferner treten die zwei inneren Rohrsegmente 100a, 100b aus der Hülse auf der Seite des inneren Endes heraus und weisen jeweils unterschiedliche Auskraglängen auf. Am zum äußeren Ende 12 der Einweg-Anschlusseinrichtung hin gerichteten Ende der zwei inneren Rohrsegmente 100a, 100b tritt jeweils einer der Füllstandsmesser 60a, 60b aus.

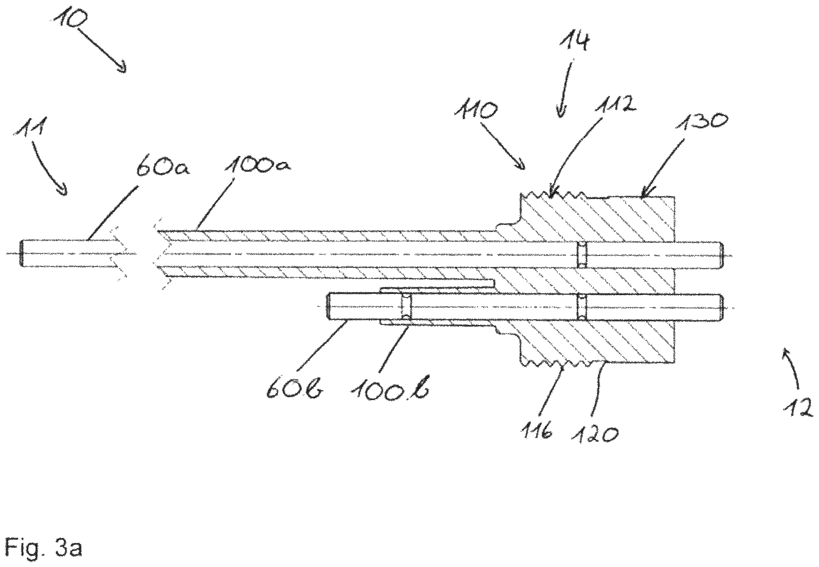

Figur 3a zeigt eine längsgeschnittene Schnittdarstellung einer Einweg-Anschlusseinrichtung gemäß Figur 1. Die Einweg-Anschlusseinrichtung 10 erstreckt sich von einem äußeren Ende 12 bis zu einem inneren Ende 11. An dem äußeren Ende 12 treten ein erster Füllstandsmesser 60a und ein zweiter Füllstandsmesser 60b aus der Einweg-Anschlusseinrichtung 10 in Längsrichtung heraus. Die Füllstandsmesser 60a, 60b sind analog zu den zuvor erfolgten Figurenbeschreibungen als Stabelektroden ausgeführt. Das äußere Ende 12 der Einweg-Anschlusseinrichtung 10 wird gebildet durch einen Kraftaufnahmeabschnitt 130, der dazu ausgebildet ist eine extern aufgebrachte Kraft aufzunehmen, mit der beispielsweise die Einweg-Anschlusseinrichtung 10 in eine Anschlusseinrichtung eingeführt werden kann.

In Längsrichtung, in Richtung des inneren Endes 11 ist an den Kraftaufnahmeabschnitt 130 ein Befestigungsabschnitt 110 angeordnet. Der Befestigungsabschnitt 110 wird gebildet durch eine Außenumfangsfläche 112, wobei diese einen kleineren Durchmesser aufweist als der Kraftaufnahmeabschnitt 130. Durch diese Durchmesserdifferenz entsteht am Übergang vom Kraftaufnahmeabschnitt 130 zum Befestigungsabschnitt 110 ein Absatz. Eine dadurch entstehende Fläche weist eine Flächennormale auf, die parallel zu einer Mittelachse des Kraftaufnahmeabschnitts 130 und des Befestigungsabschnitts 110 ausgerichtet ist. Die so entstehende Anschlagsfläche 120 dient dem Einsetzen der Einweg-Anschlusseinrichtung 10 in eine Anschlussöffnung eines Bioreaktors. Die Anschlagsfläche 120 liegt dabei auf einer Oberfläche auf, die sich im unmittelbaren Umfeld der Anschlussöffnung befindet.

Der Befestigungsabschnitt 110 weist auf der Außenumfangsfläche 112 eine Befestigungsstruktur 114 auf, die im vorliegenden Ausführungsbeispiel als Außengewinde 116 ausgeführt ist. Auf der Stirnseite des Befestigungsabschnitts 110, die dem inneren Ende 11 zugewandt ist, sind zwei innere Rohrsegment 100a angeordnet. Mittelachsen dieser zwei inneren Rohrsegmente 100a sind parallel zur Mittelachse des Kraftaufnahmeabschnitts 130 und des Befestigungsabschnitts 110. Die bezeichneten Füllstandsmesser 60a, 60b treten an den zum äußeren Ende 12 der Einweg-Anschlusseinrichtung hin gerichteten Enden des ersten inneren Rohrsegment 100a und des zweiten inneren Rohrsegment 100b aus. Die Füllstandsmesser 60a, 60b erstrecken sich demnach vollständig vom äußeren Ende 12 bis zum inneren Ende 11 der Einweg-Anschlusseinrichtung 10. Die Füllstandsmesser 60a, 60b sind dafür jeweils in Durchgänge eingeführt bzw. bereits während des Produktionsprozesses umspritzt, wobei die Durchgänge jeweils mit einer gemeinsamen Mittelachse durch die Rohrsegmente 100a, den Befestigungsabschnitt 110 und den Kraftaufnahmeabschnitt 130 hindurchführen.

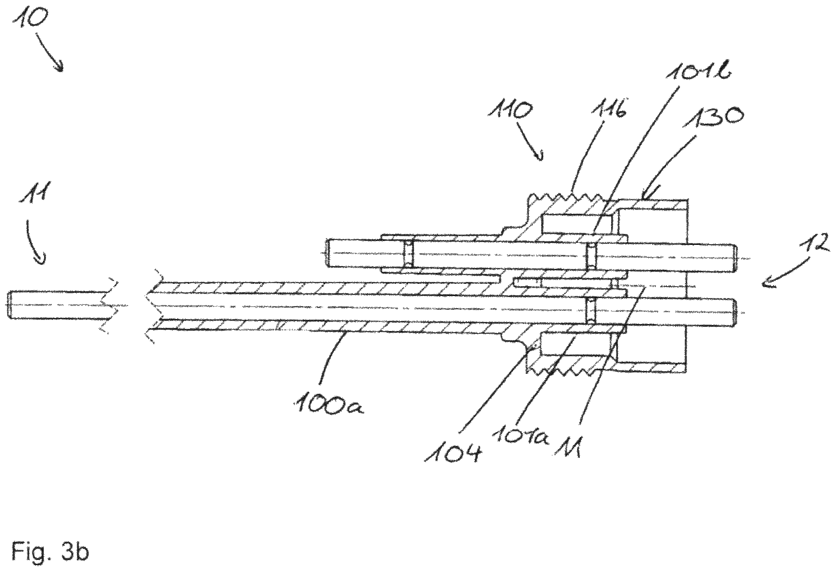

Figur 3b zeigt eine längsgeschnittene Schnittdarstellung einer möglichen Variante der Einweg-Anschlusseinrichtung 10 gemäß Figur 1, wobei die Einweg-Anschlusseinrichtung 10 hier, im Gegensatz zur Variante in Figur 3a, einen Hohlraum im Bereich eines Kraftaufnahmeabschnitts 130 und im Bereich des Befestigungsabschnitts 110 aufweist. Der Hohlraum wird in radialer Richtung von einer Mittelachse M umwandet, wobei die äußere Oberfläche dieser Umwandung im Bereich des Befestigungsabschnitts 110 ein Außengewinde 116 umfasst.

Die Begrenzungsscheibe 104 trennt, im montierten Zustand der Einweg-Anschlusseinrichtung 10, den Innenraum eines Bioreaktors von der Umgebung des Bioreaktors ab. An der zum äußeren Ende 12 gewandten Seite der Begrenzungsscheibe 104 sind ein erstes äußeres Rohrsegment 101a und ein zweites äußeres Rohrsegment 101b angeordnet. Das erste äußere Rohrsegment 101a weist den gleichen Durchgang auf wie das erste innere Rohrsegment 100a. Ferner weist das zweite äußere Rohrsegment 101b den gleichen Durchgang auf wie das zweite innere Rohrsegment 100b. Um den begrenzungsfreien Durchgang von einem inneren zu einem äußeren Rohrsegment zu gewährleisten, weist die Begrenzungsscheibe 104 Durchgangslöcher auf, deren Durchtrittsrichtung und deren Mittelachse jeweils gleich der Durchtrittsrichtung und der Mittelachse der an diesen angeordneten Rohrsegmenten entspricht.

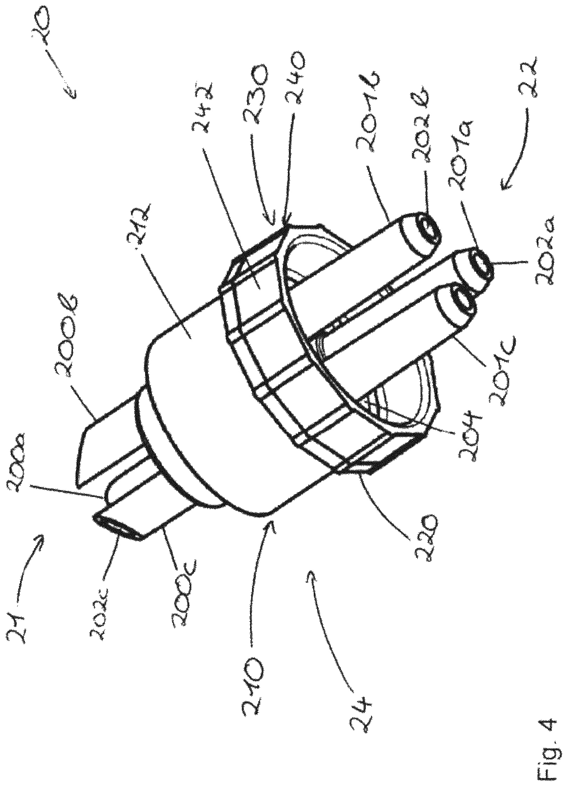

Figur 4 zeigt eine dreidimensionale Darstellung einer zweiten beispielhaften Ausführungsform einer Einweg-Anschlusseinrichtung 20. Die Einweg-Anschlusseinrichtung 20 weist ein inneres Ende 21 und ein äußeres Ende 22 auf. Das innere Ende 21 der Einweg-Anschlusseinrichtung 20 ist das Ende, das in eine Anschlussöffnung eines Bioreaktors eingeführt und/oder eingesetzt wird. Das innere Ende 21 ist demnach im eingesetzten Zustand dem Innenraum des Bioreaktors zugewandt. Das innere Ende 21 umfasst in diesem Ausführungsbeispiel ein erstes inneres Rohrsegment 200a, ein zweites inneres Rohrsegment 200b und ein drittes inneres Rohrsegment 200c. Die inneren Rohrsegmente 200a, 200b, 200c weisen jeweils die gleiche axiale Länge und darüber hinaus auch die gleiche Auskraglänge, bezogen auf eine Begrenzungsscheibe 204, auf. Die Begrenzungsscheibe 204 weist eine flächige Geometrie auf, deren Flächennormale parallel zur Durchtrittsrichtung der Rohrsegmente verläuft. Die inneren Rohrsegmente 200a, 200b, 200c weisen jeweils einen Durchgang 202a, 202b, 202c auf. Die drei inneren Rohrsegmente 200a, 200b, 200c sind ferner jeweils in radialer Richtung voneinander beabstandet.

Das äußere Ende 22 ist das Ende, das nicht in die Anschlussöffnung des Bioreaktors eingeführt und/oder eingesetzt wird. Das äußere Ende 22 ist demnach im eingesetzten Zustand dem Innenraum des Bioreaktors abgewandt. Das äußere Ende 22 umfasst drei äußere Rohrsegmente 201a, 201b, 201c, welche jeweils die gleiche axiale Länge aufweisen und in radialer Richtung voneinander beabstandet sind. Die drei äußeren Rohrsegmente weisen ferner in dieser Ausführungsvariante die gleiche Auskraglänge, bezogen auf die Begrenzungsscheibe 204, auf.

Die äußeren Rohrsegmente 201a, 201b, 201c umfassen ebenfalls jeweils die Durchgänge 202a, 202b,202c auf. Jeder einzelne Durchgang 202a, 202b, 202c verläuft vollständig durch die Einweg-Anschlusseinrichtung hindurch, so dass ein Durchgang 202a, 202b,202c am inneren Ende 21, in einem inneren Rohrsegment 200a, 200b, 200c beginnt und vorzugsweise ununterbrochen bis zum äußeren Ende 22 in einem äußeren Rohrsegment 201a, 201b, 201c verläuft. Der Durchgang 202a wird demnach überwiegend durch das erste innere Rohrsegment 200a und das erste äußere Rohrsegment 201a gebildet. Der Durchgang 202b wird ferner überwiegend durch das zweite innere Rohrsegment 200b und das zweite äußere Rohrsegment 201b gebildet. Der Durchgang 202c wird ferner überwiegend durch das dritte innere Rohrsegment 200c und das dritte äußere Rohrsegment 201c gebildet.

Ein einzelnes inneres Rohrsegment 200a, 200b, 200c und ein korrespondierendes einzelnes äußeres Rohrsegment 201a, 201b, 201c haben demnach eine gemeinsame Mittelachse. Ein inneres Rohrsegment 200a, 200b, 200c und ein korrespondierendes äußeres Rohrsegment 201a, 201b, 201c mit gemeinsamer Mittelachse werden durch die orthogonal zur Mittelachse angeordnete kreisförmige Begrenzungsscheibe 204 in einer definierten Position gehalten. Die kreisförmige Scheibe 204 weist Durchtrittsöffnungen auf, so dass die Durchgänge 202a, 202b,202c vom inneren Ende 21 bis zum äußeren Ende 22 durchgängig sind. Die Enden der inneren Rohrsegmente 200a, 200b, 200c weisen jeweils eine Stirnfläche auf, die ca. 45 Grad zur Mittelachse des Rohrsegments geneigt ist. Die Wandstärke der äußeren Rohrsegmente 201a, 201b, 201c nimmt zum äußeren Ende hin ab, wobei sich die Wandstärke in etwa halbiert.

Die inneren und äußeren Rohrsegmente sowie die Begrenzungsscheibe 204 werden in radialer Richtung von einer Hülse 24 umgeben, die einen Befestigungsabschnitt 210, eine Anschlagsfläche 220 und einen Kraftaufnahmeabschnitt 230 aufweist. Der Befestigungsabschnitt 210 weist eine Außenumfangsfläche 212 auf. Die Außenumfangsfläche 212 weist ferner einen im Wesentlichen konstanten Durchmesser auf. Eine Befestigungsstruktur 214 ist hier in Form eines derart bemessenen Außendurchmessers des Befestigungsabschnitts 210 realisiert, dass eine Klemmverbindung bzw. ein Presssitz der Einweg-Anschlusseinrichtung 20 in einer Anschlussöffnung realisiert wird. Der Kraftaufnahmeabschnitt 230 ist unmittelbar an das Ende des Befestigungsabschnitts 210, das zum äußeren Ende 22 zugewandt ist, angeordnet. Der Kraftaufnahmeabschnitt 230 weist einen kreisrunden Innendurchmesser auf, der größer ist als der Innendurchmesser des Befestigungsabschnitts 210.

Die äußere Umfangsfläche des Kraftaufnahmeabschnitts 230 weist ein Zwölfkantprofil 240 auf. Das Zwölfkantprofil 240 besteht aus zwölf ebenen, gleich großen Flächen 242, die auf der äußeren Umfangsfläche des Kraftaufnahmeabschnitts 230 gleichmäßig verteilt sind. Die einzelnen Flächen 142 sind derart angeordnet, dass deren jeweilige Flächennormale durch die Mittelachse der Hülse 24 verläuft und jeweils zwei gegenüberliegende Kanten einer einzelnen Fläche wiederum an je eine Kante von zwei weiteren Flächen angrenzen.

Dadurch dass der Kraftaufnahmeabschnitt 230 einen größeren Durchmesser aufweist als der Befestigungsabschnitt 210 entsteht am Übergang vom Befestigungsabschnitt 210 zum Kraftaufnahmeabschnitt 230 ein Absatz bzw. eine Stufe, die orthogonal zur Oberfläche des Befestigungsabschnitts 210 ausgerichtet ist. Eine Anschlussöffnung eines Bioreaktors ist vorzugsweise derart gestaltet, dass der Durchmesser der Anschlussöffnung eine gleiche oder eine kleinere Abmessung aufweist als der Durchmesser des Befestigungsabschnitts 210. Ferner ist der Durchmesser der Anschlussöffnung kleiner als der Durchmesser des Kraftaufnahmeabschnitts 230, so dass die vorbezeichnete Stufe als Anschlagsfläche 220 wirkt. Die Anschlagsfläche 220 liegt nach dem Einsetzen der Einweg-Anschlusseinrichtung 20 auf der Oberfläche des Elements des Bioreaktors auf, das die Anschlussöffnung aufweist.

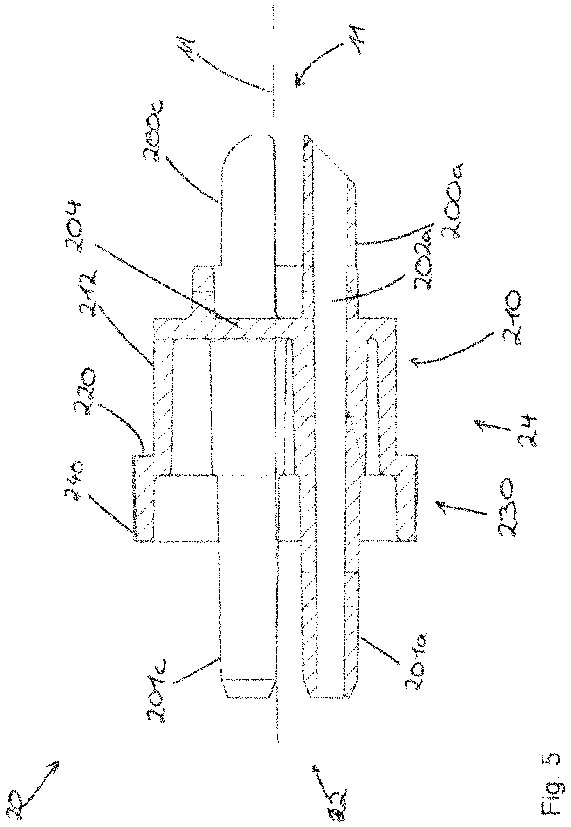

Figur 5 zeigt eine längsgeschnittene Schnittdarstellung einer Ausführungsvariante der Einweg-Anschlusseinrichtung gemäß Figur 4. Die Einweg-Anschlusseinrichtung 20 weist am äußeren Ende 22 insgesamt drei äußere Rohrsegmente auf, wobei das Rohrsegment 201b außerhalb der Schnittebene angeordnet ist und das Rohrsegment 201c in ungeschnittener Perspektive dargestellt ist, da dieses hinter der Schnittebene liegt. Die Einweg-Anschlusseinrichtung 20 weist ferner eine Hülse 24 auf, die den Kraftaufnahmeabschnitt 230, die Anschlagsfläche 220 und den Befestigungsabschnitt 210 umfasst.

Der Befestigungsabschnitt 210 weist eine rotationssymmetrische Form bezüglich der Mittelachse M auf, so dass der Befestigungsabschnitt 210 ebenfalls die Form einer Hülse aufweist. Ferner umfasst der Befestigungsabschnitt 210 eine Außenumfangsfläche 212, an der eine nicht dargestellte Befestigungsstruktur angebracht werden kann. An der dem inneren Ende 21 zugewandten Stirnseite des Befestigungsabschnitts 210 befindet sich eine Begrenzungsscheibe 204, die die Stirnseite des Befestigungsabschnitts 210 im Wesentlichen vollständig abschließt. Die Begrenzungsscheibe 204 weist daher eine Flächennormale auf, die parallel zur Mittelachse M ausgerichtet ist.

Unter Beachtung der Figur 4 und der Figur 5 sind an der Begrenzungsscheibe 204 ferner die äußeren Rohrsegmente 201a, 201b, 201c und die inneren Rohrsegmente 200a, 200b, 200c angeordnet. Dabei bilden jeweils ein äußeres Rohrsegment und ein inneres Rohrsegment einen gemeinsamen Durchgang 202a, 202b oder 202c. An der Stelle an dem ein äußeres Rohrsegment und ein inneres Rohrsegment an der Begrenzungsscheibe 204 angeordnet ist, befindet sich in der Begrenzungsscheibe 204 ebenfalls eine Durchgangsöffnung, so dass jeder der drei Durchgänge 202a, 202b, 202c vom inneren Ende 21 bis zum äußeren Ende 22 durchgängig ist.

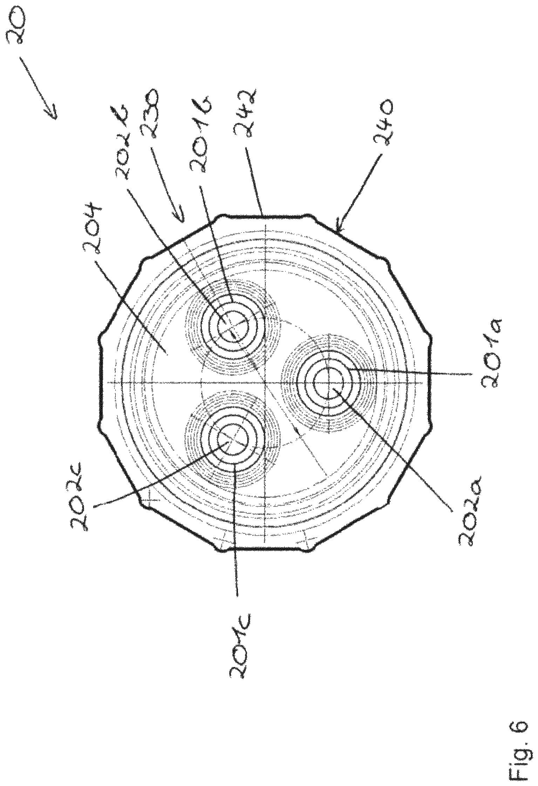

Figur 6 zeigt eine Draufsicht auf die Einweg-Anschlusseinrichtung 20 gemäß Figur 4. Drei äußere Rohrsegmente 201a, 201b, 201c sind an der Begrenzungsscheibe 204 angeordnet. Die äußeren Rohrsegmente 201a, 201b, 201c weisen ferner einen kreisrunden Querschnitt auf, wobei deren Mittelpunkte jeweils den gleichen Abstand zum Mittelpunkt der Begrenzungsscheibe 204 aufweisen. Darüber hinaus weisen die drei äußeren Rohrsegmente die zuvor bezeichneten Durchgänge 202a, 202b oder 202c auf. Die Einweg-Anschlusseinrichtung 20 umfasst ferner einen Kraftaufnahmeabschnitt 230 auf, der dazu ausgebildet ist mit einer radialen und/oder axialen Kraft beaufschlagt zu werden, um die Einweg-Anschlusseinrichtung 20 vorzugsweise in und/oder an einer Anschlussöffnung eines Bioreaktors zu befestigen. Der Kraftaufnahmeabschnitt 230 weist daher das bereits zuvor beschriebene Mehrkantprofil auf, das hier im Wesentlichen aus zwölf Mehrkantprofilflächen 242 besteht.

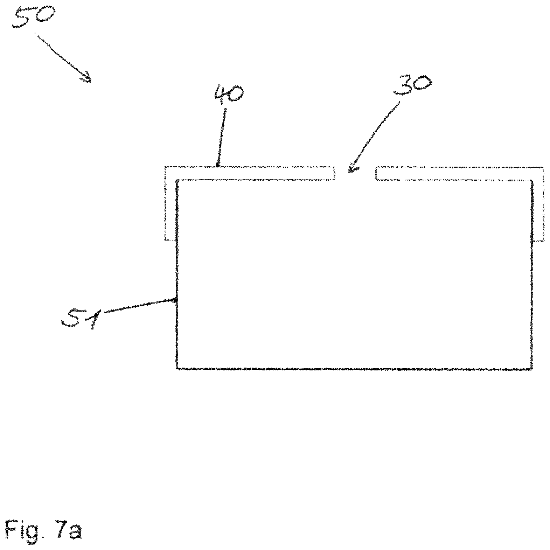

Figur 7a zeigt eine schematische Darstellung eines Bioreaktors 50 mit Kopfplatte 40. Der Bioreaktor 50 umfasst einen Bioreaktorbehälter 51, der vorzugsweise mit Ausnahme von einer Seite vollständig umwandet ist. Der Behälter ist vorzugsweise aus einem metallischen Material, vorzugsweise rostfreier Stahl, und/oder Kunststoff ausgebildet oder umfasst diese Materialien. Die nicht umwandete Seite des Bioreaktorbehälters 51 ist von einer Kopfplatte 40 verschlossen. Die Kopfplatte weist ferner eine Anschlussöffnung 30 auf, die dazu ausgebildet ist, eine erfindungsgemäße Einweg-Anschlusseinrichtung 10, 20 aufzunehmen. Vorzugsweise weist die Anschlussöffnung 30 an ihrer Innenumfangsfläche ein Innengewinde auf, das die Aufnahme eines komplementär ausgebildeten Außengewindes an einer Einweg-Anschlusseinrichtung 10, 20 ermöglicht.

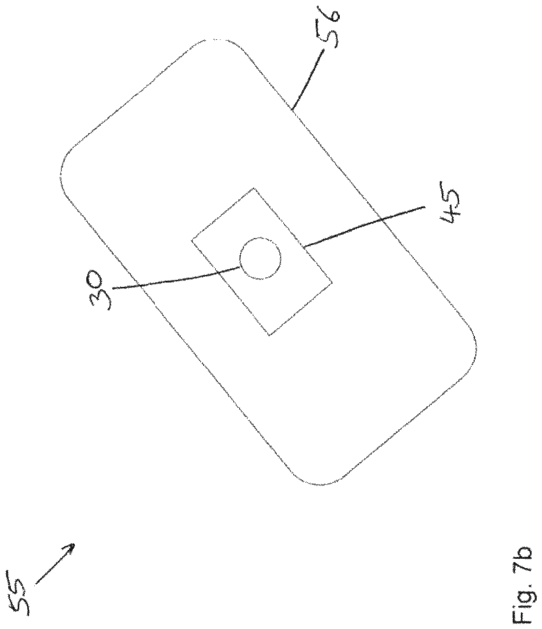

Figur 7b zeigt eine schematische Darstellung eines Beutel-Bioreaktors 55 mit einem Anschlusselement 45 und einem im Wesentlichen oder zumindest abschnittsweise nicht formstabilen Beutel 56. Das Anschlusselement 45 ist an dem Beutel 56 derart angeordnet, dass die Trennfuge zwischen Anschlusselement 45 und Beutel 56 eine vorzugsweise hermetische Abdichtung des Innenraums des Beutels 56 von der Umgebung des Beutel-Bioreaktors 55 gewährleisten kann. Ferner weist das Anschlusselement 45 eine Anschlussöffnung 30 auf, die im vorliegenden Ausführungsbeispiel einen kreisrunden Querschnitt aufweist. Die Anschlussöffnung 30 ist dazu ausgebildet eine erfindungsgemäße Einweg-Anschlusseinrichtung 10, 20 aufzunehmen. Vorzugsweise weist die Anschlussöffnung 30 an ihrer Innenumfangsfläche ein Innengewinde auf, das die Aufnahme eines komplementär ausgebildeten Außengewinde an einer Einweg-Anschlusseinrichtung 10, 20 ermöglicht.

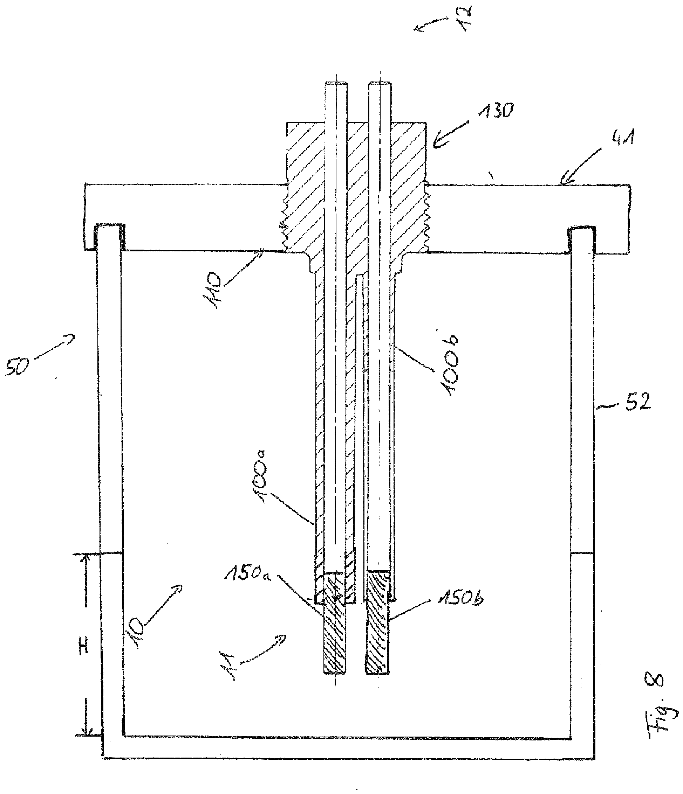

Figur 8 zeigt eine längsgeschnittene Schnittdarstellung einer weiteren beispielhaften Ausgestaltung einer Einweg-Anschlusseinrichtung 10, die in einer Kopfplatte 41 eines Bioreaktors 50 angeordnet ist. Der Bioreaktor 50 umfasst weiter einen Bioreaktorbehälter 52, an dem die Kopfplatte befestigt ist. Die Einweg-Anschlusseinrichtung 10 erstreckt sich von einem äußeren Ende 12 bis zu einem inneren Ende 11, das in den Bioreaktorbehälter 52 und damit in den Bioreaktor 50 ragt. Das äußere Ende 12 der Einweg-Anschlusseinrichtung 10 wird gebildet durch einen Kraftaufnahmeabschnitt 130, der dazu ausgebildet ist, extern aufgebrachte Kräfte. Solche Kräfte können insbesondere aus dem Einbringvorgang (beispielsweise durch Einschrauben) der Einweg-Anschlusseinrichtung 10 in die Kopfplatte 41 resultieren, etwa aus einem Drehmoment, dass über Kraftaufnahmeabschnitt 130 aufgebracht werden kann.

In Längsrichtung, in Richtung des inneren Endes 11 ist an den Kraftaufnahmeabschnitt 130 ein Befestigungsabschnitt 110 mit einem Außengewinde angeordnet. Auf der Stirnseite des Befestigungsabschnitts 110, die dem inneren Ende 11 zugewandt ist, sind zwei innere Rohrsegment 100a, 100b aus Kunststoff angeordnet. Die Mittelachsen dieser zwei inneren Rohrsegmente 100a, 100b sind parallel zur Mittelachse des Kraftaufnahmeabschnitts 130 und des Befestigungsabschnitts 110 angeordnet. An den Enden der inneren Rohrsegmente 100a, 100b ist jeweils ein Filterelement 150a, 150b angeordnet. Im gezeigten Ausführungsbeispiel handelt es sich um keramische Filterelemente Diese keramischen Filterelemente 150a, 150b ragen in der gezeigten Ausführungsform über das innere Ende des jeweiligen Rohrsegmentes hinaus. Die inneren Rohrsegmente 100a, 100b können sowohl für eine Medien- oder Gaszufuhr als auch für einen Medienabzug oder eine Medienableitung genutzt werden. Die keramischen Filterelemente sorgen dafür, dass keine Zellen aus der Kulturbrühe bei einer Verwendung des jeweiligen Rohrsegmentes für eine Medienableitung den Bioreaktor verlassen, da diese vom Filter aufgehalten werden. Andererseits kann über die keramischen Filter auch eine feinblasige Verteilung des zugeführten Gases oder des zugeführten Mediums erreicht werden.

Die Rohrsegmente 100a, 100b sind dabei ausreichend lang ausgebildet, dass ihr jeweiliges inneres Ende sich unterhalb einer Mindestfüllhöhe H für Kulturmedium des Bioreaktors befindet. Die Rohrsegmente 100a, 100b umfassen vorzugsweise die keramischen Filterelemente 150a, 150b. Daher kann es auch ausreichen, wenn sich nur die Filterelemente 150a, 150b bzw. deren - im Betriebszustand unteren - Enden unterhalb der Mindestfüllhöhe H befinden.

Damit wird sichergestellt, dass die Medienzuführung direkt in das Kulturmedium erfolgen kann. Auch eine Verwendung zur Medienabführung erfolgt vorteilhaft, wenn das Rohrsegment in das Kulturmedium ragt. Um dies unabhängig von der Füllhöhe bei ordnungsgemäßer Verwendung sicherzustellen, liegt das innere Ende der Rohrsegmente 100a, 100b unterhalb der Mindestfüllhöhe H. So ist selbst bei minimaler Befüllung eine Medienzufuhr oder -abfuhr direkt ins oder direkt aus dem Kulturmedium gewährleistet.

### Bezugszeichen

- 10, 20: Einweg-Anschlusseinrichtung
- 11, 21: Inneres Ende
- 12, 22: Äußeres Ende
- 14, 24: Hülse
- 30: Anschlussöffnung
- 40, 41: Kopfplatte
- 45: Anschlusselement
- 50: Bioreaktor
- 51, 52: Bioreaktorbehälter
- 55: Beutel-Bioreaktor
- 60a: erster als Füllstandsmesser ausgebildeter Sensor/ Stabelektrode
- 60b: zweiter als Füllstandsmesser ausgebildeter Sensor / Stabelektrode
- 100a, 200a: erstes inneres Rohrsegment
- 100b, 200b: zweites inneres Rohrsegment
- 200c: drittes inneres Rohrsegment
- 101a, 201a: erstes äußeres Rohrsegment
- 101b, 201b: zweites äußeres Rohrsegment
- 201c: drittes äußeres Rohrsegment
- 102a, 202a: erster Durchgang
- 102b, 202b: zweiter Durchgang
- 102c, 202c: dritter Durchgang
- 104, 204: Begrenzungsscheibe
- 110, 210: Befestigungsabschnitt
- 112, 212: Außenumfangsfläche
- 114, 214: Befestigungsstruktur
- 116, 216: Außengewinde
- 120, 220: Anschlagsfläche
- 130, 230: Kraftaufnahmeabschnitt
- 140, 240: Mehrkantprofil
- 150a, 150b: Filterelement
- M: Mittelachse (der Außenumfangsfläche)
- H: Mindestfüllhöhe

## Patentansprüche

1. Kopfplatte für einen Bioreaktor, wobei die Kopfplatte dazu ausgebildet ist, an einem Behälter des Bioreaktors befestigt zu werden,
die Kopfplatte umfassend
eine Anschlussöffnung und
eine in die Anschlussöffnung eingesetzte Einweg-Anschlusseinrichtung (10), **dadurch gekennzeichnet, dass** die Einweg-Anschlusseinrichtung (10) mehrere Durchgänge (102) und einen Befestigungsabschnitt (110), aufweist,
und die Einweg-Anschlusseinrichtung (10) einstückig und aus Kunststoff ausgebildet ist und der Befestigungsabschnitt (110) mit einer an einer Außenumfangsfläche (112) ausgebildeten Befestigungsstruktur (114) in der Anschlussöffnung befestigt ist.

2. Kopfplatte nach Anspruch 1,
**dadurch gekennzeichnet, dass** die Befestigungsstruktur in Form eines Außengewindes (116) und/oder in Form von Rippen und/oder Vorsprüngen und/oder Schnappelementen und/oder Rastelementen und/oder Vertiefungen ausgebildet ist.

3. Kopfplatte nach mindestens einem der vorhergehenden Ansprüche,
**dadurch gekennzeichnet, dass** der Befestigungsabschnitt (110) eine umlaufende Anschlagsfläche (120) aufweist, welche sich von der Außenumfangsfläche (112) in radialer Richtung nach außen erstreckt und/odereinen Kraftaufnahmeabschnitt (130) aufweist, welcher ein Mehrkantprofil (140) umfasst und sich vorzugsweise unmittelbar an die Anschlagsfläche (120) anschließt.

4. Kopfplatte nach mindestens einem der vorhergehenden Ansprüche,
**dadurch gekennzeichnet, dass** ein Durchgang (102), mehrere oder sämtliche Durchgänge (102) zumindest abschnittsweise als Rohrsegment (100) ausgebildet sind, wobei sich vorzugsweise ein Rohrsegment (100), mehrere oder sämtliche Rohrsegmente (100) parallel zu einer Mittelachse (M) der Außenumfangsfläche (112) in eine erste Richtung von der Außenumfangsfläche (112) und/oder in eine zweite Richtung von der Außenumfangsfläche (112) erstrecken, wobei vorzugsweise ein Rohrsegment (100), mehrere oder sämtliche Rohrsegmente (100) eine oder mehrere Sollbruchstellen aufweisen.

5. Kopfplatte nach Anspruch 4,
**dadurch gekennzeichnet, dass** ein Rohrsegment (100), mehrere oder sämtliche Rohrsegmente (100) unter Wärmeeinwirkung zumindest derart verformbar sind, dass der jeweilige Durchgang (102) durch Formveränderung fluiddicht verschließbar ist oder dass
ein Rohrsegment (100), mehrere oder sämtliche Rohrsegmente (100) an zumindest einem Ende mit einer Schlauchsicherungseinrichtung abschließen.

6. Kopfplatte nach mindestens einem der vorhergehenden Ansprüche 4 oder 5,
**dadurch gekennzeichnet, dass** mindestens ein Rohrsegment (100) aus Kunststoff gebildet ist und an einem zur Einbringung in den Bioreaktor vorgesehenen Ende ein Filterelement (150a, 150b) aufweist.

7. Kopfplatte nach Anspruch 6,
**dadurch gekennzeichnet, dass** das Filterelement (150a, 150b) eine Porengröße in einem Bereich von 0,1 µm - 500 µm, insbesondere von 0,1 - 0,2 µm, von 0,1 bis 1 µm, von 0,5 - 5 µm, von 5 - 250 µm oder von 5 - 500 µm aufweist, und/oder **dadurch gekennzeichnet, dass** das Filterelement (150a, 150b) eine Membran enthält und/oder eine poröse Struktur aufweist, und/oder
**dadurch gekennzeichnet, dass** das Filterelement (150a, 150b) Glasfilter, eine poröse Keramik, insbesondere eine Schaumkeramik, einen Kunststofffilter oder eine Filterkassette umfasst oder daraus besteht.

8. Kopfplatte nach mindestens einem der vorgehenden Ansprüche 6 oder 7,
**dadurch gekennzeichnet, dass** der Kunststoff des mindestens einen Rohrsegments ein thermoplastischer Kunststoff ist und das Filterelement Aluminiumoxid und/oder Zirkonoxid und/oder Borosilikat und/oder Polycarbonat und/oder Polypropylen und/oder Polyethylen, aufweist.

9. Kopfplatte nach mindestens einem der vorhergehenden Ansprüche,
**gekennzeichnet durch** einen oder mehrere Sensoren (60) und/oder Elektroden, wobei vorzugsweise in einem Durchgang (102), mehreren oder sämtlichen Durchgängen (100) mindestens ein Sensor (60) und/oder mindestens eine Elektrode angeordnet ist.

10. Bioreaktor zur Kultivierung von Mikroorganismen und/oder Zellkulturen, mit einem Behälter und einer Kopfplatte zum Verschließen des Behälters,
**dadurch gekennzeichnet, dass** die Kopfplatte nach mindestens einem der vorhergehenden Ansprüche ausgebildet ist.

11. Bioreaktor nach Anspruch 10,
**dadurch gekennzeichnet, dass** die Anschlusseinrichtung (10) mindestens ein Rohrsegment umfasst, dessen eines im Bioreaktor angeordnetes Ende sich unterhalb einer Mindestfüllhöhe für Kulturmedium des Bioreaktors befindet.

12. Anschlusselement für einen Beutel-Bioreaktor, wobei das Anschlusselement dazu ausgebildet ist, mit einem Beutelbehälter verbunden zu werden und/oder einstückig mit diesem ausgebildet zu werden, das Anschlusselement umfassend eine Anschlussöffnung, und
eine in die Anschlussöffnung eingesetzte Einweg-Anschlusseinrichtung (10), **dadurch gekennzeichnet, dass** die Einweg-Anschlusseinrichtung (10) mehrere Durchgänge (102) und einen Befestigungsabschnitt (110), aufweist,
und die Einweg-Anschlusseinrichtung (10) einstückig und aus Kunststoff ausgebildet ist und der Befestigungsabschnitt (110) mit einer an einer Außenumfangsfläche (112) ausgebildeten Befestigungsstruktur (114) in der Anschlussöffnung befestigt ist.

13. Beutel-Bioreaktor zur Kultivierung von Mikroorganismen und/oder Zellkulturen, mit einem verformbaren Beutelbehälter mit einem Anschlusselement,
**dadurch gekennzeichnet, dass** das Anschlusselement nach Anspruch 12 ausgebildet ist.

14. Beutel-Bioreaktor nach Anspruch 13,
**dadurch gekennzeichnet, dass** die Anschlusseinrichtung (10) des Anschlusselementes mindestens ein Rohrsegment umfasst, dessen eines im Beutel-Bioreaktor angeordnetes Ende sich unterhalb einer Mindestfüllhöhe für Kulturmedium des Beutel-Bioreaktors befindet.

## Claims

1. A top plate for a bioreactor, wherein the top plate is designed to be fastened to a vessel of the bioreactor,
the top plate comprising
a connection opening, and
a single-use connection device (10) inserted into the connection opening, **characterized in that** the single-use connection device (10) comprises multiple passages (102) and a fastening section (110), and
the single-use connection device (10) is formed in one piece and from plastic, and the fastening section (110) is fastened in the connection opening by a fastening structure (114) on an outer circumferential surface (112).

2. The top plate as claimed in claim 1,
**characterized in that** the fastening structure is realized in the form of an external thread (116) and/or in the form of ribs and/or projections and/or snap-action elements and/or detent elements and/or depressions.

3. The top plate as claimed in at least one of the preceding claims,
**characterized in that** the fastening section (110) has an encircling stop surface (120), which extends outward in a radial direction from the outer circumferential surface (112), and/or has a force admission section (130), which comprises a polygonal profile (140) and which preferably directly adjoins the stop surface (120).

4. The top plate as claimed in at least one of the preceding claims,
**characterized in that** one passage (102), multiple or all passages (102) are formed at least in sections as a pipe segment (100), wherein, preferably, one pipe segment (100), multiple or all pipe segments (100) extend parallel to a central axis (M) of the outer circumferential surface (112) in a first direction from the outer circumferential surface (112) and/or in a second direction from the outer circumferential surface (112), wherein, preferably, one pipe segment (100), multiple or all pipe segments (100) have one or more predetermined breaking points.

5. The top plate as claimed in claim 4,
**characterized in that** one pipe segment (100), multiple or all pipe segments (100) are at least deformable under the action of heat such that the respective passage (102) is closable in fluid-tight fashion as a result of a change in shape, or **in that** one pipe segment (100), multiple or all pipe segments (100) are closed off at at least one end by means of a hose-securing device.

6. The top plate as claimed in at least one of the preceding claims 4 and 5, **characterized in that** at least one pipe segment (100) is formed from plastic and has a filter element (150a, 150b) at an end provided for insertion into the bioreactor.

7. The single top plate as claimed in claim 6,
**characterized in that** the filter element (150a, 150b) has a pore size in a range from 0.1 µm - 500 µm, in particular from 0.1 - 0.2 µm, from 0.1 to 1 µm, from 0.5-5 µm, from 5 - 250 µm or from 5 - 500 µm, and/or
**characterized in that** the filter element (150a, 150b) comprises a diaphragm and/or has a porous structure, and/or
**characterized in that** the filter element (150a, 150b) comprises or is composed of glass filter, a porous ceramic, in particular a foamed ceramic, a plastics filter or a filter cassette.

8. The top plate as claimed in at least one of the preceding claims 6 and 7, **characterized in that** the plastic of the at least one pipe segment is a thermoplastic, and the filter element has aluminum oxide and/or zirconium oxide and/or borosilicate and/or polycarbonate and/or polypropylene and/or polyethylene.

9. The top plate as claimed in at least one of the preceding claims,
**characterized by** one or more sensors (60) and/or electrodes, wherein, preferably, at least one sensor (60) and/or at least one electrode is arranged in one passage (102), multiple or all passages (100).

10. A bioreactor for cultivating microorganisms and/or cell cultures, having a vessel and a top plate for closing off the vessel,
**characterized in that** the top plate is designed as claimed in at least one of the preceding claims.

11. The bioreactor as claimed in claim 10,
**characterized in that** the connection device (10) comprises at least one pipe segment whose end arranged in the bioreactor is situated below a minimum fill level for culture medium of the bioreactor.

12. A connection element for a pouch bioreactor, wherein the connection element is designed to be connected to, and/or to be formed in one piece with, a pouch vessel, the connection element comprising
a connection opening, and
a single-use connection device (10) inserted into the connection opening, **characterized in that** the single-use connection device (10) comprises multiple passages (102) and a fastening section (110), and
the single-use connection device (10) is formed in one piece and from plastic, and the fastening section (110) is fastened in the connection opening by a fastening structure (114) on an outer circumferential surface (112).

13. A pouch bioreactor for cultivating microorganisms and/or cell cultures, having a deformable pouch vessel with a connection element,
**characterized in that** the connection element is designed as claimed in claim 12.

14. The pouch bioreactor as claimed in claim 13,
**characterized in that** the connection device (10) of the connection element comprises at least one pipe segment whose end arranged in the pouch bioreactor is situated below a minimum fill level for culture medium of the pouch bioreactor.

## Revendications

1. Plaque supérieure pour un bioréacteur, dans laquelle la plaque supérieure est réalisée afin d'être fixée à un récipient du bioréacteur,
la plaque supérieure comprenant
une ouverture de raccordement et
un dispositif de raccordement à usage unique (10) inséré dans l'ouverture de raccordement,
**caractérisée en ce que** le dispositif de raccordement à usage unique (10) présente plusieurs passages (102) et une section de fixation (110),
et le dispositif de raccordement à usage unique (10) est réalisé d'un seul tenant et en matière plastique et la section de fixation (110) est fixée à une structure de fixation (114) réalisée au niveau d'une surface périphérique extérieure (112) dans l'ouverture de raccordement.

2. Plaque supérieure selon la revendication 1,
**caractérisée en ce que** la structure de fixation est réalisée sous la forme d'un filet extérieur (116) et/ou sous la forme de nervures et/ou de saillies et/ou d'éléments à déclic et/ou d'éléments d'encliquetage et/ou de cavités.

3. Plaque supérieure selon au moins l'une des revendications précédentes,
**caractérisée en ce que** la section de fixation (110) présente une surface de butée (120) périphérique qui s'étend de la surface périphérique extérieure (112) dans la direction radiale vers l'extérieur et/ou présente une section de réception de force (130) qui comporte un profil polygonal (140) et est contigüe de préférence directement à la surface de butée (120).

4. Plaque supérieure selon au moins l'une des revendications précédentes,
**caractérisée en ce qu'**un passage (102), plusieurs ou tous les passages (102) sont réalisés au moins par section comme segment tubulaire (100), dans laquelle de préférence un segment tubulaire (100), plusieurs ou tous les segments tubulaires (100) s'étendent parallèlement à un axe médian (M) de la surface périphérique extérieure (112) dans une première direction de la surface périphérique extérieure (112) et/ou dans une seconde direction de la surface périphérique extérieure (112), dans laquelle de préférence un segment tubulaire (100), plusieurs ou tous les segments tubulaires (100) présentent un ou plusieurs points destinés à la rupture.

5. Plaque supérieure selon la revendication 4,
**caractérisée en ce qu'**un segment tubulaire (100), plusieurs ou tous les segments tubulaires (100) sont déformables sous l'effet de la chaleur au moins de telle manière que le passage concerné (102) soit refermable par modification de forme de manière étanche au fluide ou que
un segment tubulaire (100), plusieurs ou tous les segments tubulaires (100) se terminent à au moins une extrémité par un dispositif de fixation de tuyau.

6. Plaque supérieure selon au moins l'une des revendications précédentes 4 ou 5,
**caractérisée en ce qu'**au moins un segment tubulaire (100) est formé en matière plastique et présente à une extrémité prévue pour l'introduction dans le bioréacteur un élément de filtre (150a, 150b).

7. Plaque supérieure selon la revendication 6,
**caractérisée en ce que** l'élément de filtre (150a, 150b) présente une grandeur de pore dans une plage de 0,1 µm à 500 µm, en particulier de 0,1 à 0,2 µm, de 0,1 à 1 µm, de 0,5 à 5 µm, de 5 à 250 µm ou de 5 à 500 µm, et/ou
**caractérisée en ce que** l'élément de filtre (150a, 150b) contient une membrane et/ou présente une structure poreuse, et/ou
**caractérisée en ce que** l'élément de filtre (150a, 150b) comporte un filtre de verre, une céramique poreuse, en particulier une céramique expansée, un filtre en matière plastique ou une cassette filtrante ou s'en compose.

8. Plaque supérieure selon au moins l'une des revendications précédentes 6 ou 7,
**caractérisée en ce que** la matière plastique de l'au moins un segment tubulaire est une matière plastique thermoplastique et l'élément de filtre présente de l'oxyde d'aluminium et/ou de l'oxyde de zirconium et/ou du borosilicate et/ou du polycarbonate et/ou du polypropylène et/ou du polyéthylène.

9. Plaque supérieure selon au moins l'une des revendications précédentes,
**caractérisée par** un ou plusieurs capteurs (60) et/ou électrodes, dans laquelle de préférence dans un passage (102), plusieurs ou tous les passages (100), au moins un capteur (60) et/ou au moins une électrode est agencée.

10. Bioréacteur pour la culture de micro-organismes et/ou de cultures cellulaires, avec un récipient et une plaque supérieure pour la fermeture du récipient,
**caractérisé en ce que** la plaque supérieure est réalisée selon au moins une des revendications précédentes.

11. Bioréacteur selon la revendication 10,
**caractérisé en ce que** le dispositif de raccordement (10) comporte au moins un segment tubulaire, dont une extrémité agencée dans le bioréacteur se trouve en dessous d'une hauteur de remplissage minimale pour un milieu de culture du bioréacteur.

12. Élément de raccordement pour un bioréacteur à poche, dans lequel l'élément de raccordement est réalisé afin d'être relié à un récipient de poche et/ou d'être réalisé d'un seul tenant avec celui-ci, l'élément de raccordement comprenant
une ouverture de raccordement, et
un dispositif de raccordement à usage unique (10) inséré dans l'ouverture de raccordement,
**caractérisé en ce que** le dispositif de raccordement à usage unique (10) présente plusieurs passages (102) et une section de fixation (110),
et le dispositif de raccordement à usage unique (10) est réalisé d'un seul tenant et en matière plastique et la section de fixation (110) est fixée à une structure de fixation (114) réalisée au niveau d'une surface périphérique extérieure (112) dans l'ouverture de raccordement.

13. Bioréacteur à poche pour la culture de micro-organismes et/ou de cultures cellulaires, avec un récipient de poche déformable avec un élément de raccordement,
**caractérisé en ce que** l'élément de raccordement est réalisé selon la revendication 12.

14. Bioréacteur à poche selon la revendication 13,
**caractérisé en ce que** le dispositif de raccordement (10) de l'élément de raccordement comporte au moins un segment tubulaire, dont une extrémité agencée dans le bioréacteur à poche se trouve en dessous d'une hauteur de remplissage minimale pour le milieu de culture du bioréacteur à poche.
